# EUROPEAN PATENT APPLICATION

(11) **EP 3 882 246 A1**
(43) Date of publication of application: **22.09.2021**
(21) Application number: 19885506.6
(22) Date of filing: 12.11.2019
(51) Int. Cl.: C07D 417/14, A61K 31/427, A61K 31/4439, A61K 31/437, A61K 31/4709, A61P 31/04

(54) **MONOBACTAM COMPOUNDS AND USE THEREFOR**

(30) Priority: 13.11.2018 CN 201811348908
(71) Applicant: Nanjing Sanhome Pharmaceutical Co., Ltd., Nanjing Jiangsu 210038 (CN)
(72) Inventor: WANG, Yong, Nanjing, Jiangsu 210038 (CN); ZHAO, Liwen, Nanjing, Jiangsu 210038 (CN); WANG, Yazhou, Nanjing, Jiangsu 210038 (CN); QUAN, Xu, Nanjing, Jiangsu 210038 (CN); WANG, Xiaowei, Nanjing, Jiangsu 210038 (CN); ZHANG, Xiaoping, Nanjing, Jiangsu 210038 (CN); LV, Kunzhi, Nanjing, Jiangsu 210038 (CN); LIN, Zeqi, Nanjing, Jiangsu 210038 (CN); ZHANG, Jian, Nanjing, Jiangsu 210038 (CN); XU, Tao, Nanjing, Jiangsu 210038 (CN); CHANG, Yujie, Nanjing Economic And Technological Development Zone Jiangsu 210038 (CN)
(74) Representative: Høiberg P/S
(86) International application number: PCT/CN2019/117472
(87) International publication number: WO 2020/098635

(57) **Abstract**

Monobactam compounds and a use therefor. Specifically provided are chemical compounds represented by formula (I) or isomers, pharmaceutically acceptable salts, solvates, crystals, or prodrugs thereof, preparation methods therefor, pharmaceutical compositions containing said compounds, and a use of said compounds or compositions in treating bacterial infection. The present compounds feature excellent antibacterial activity, and have great hopes of becoming a therapeutic agent for bacterial infection.

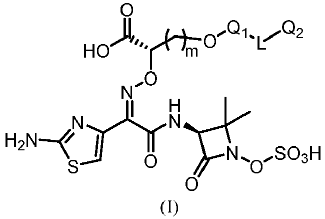

## Description

### Technical Field

The present invention belongs to the field of medicinal chemistry, and specifically relates to a class of monocyclic lactam compounds or isomers, pharmaceutically acceptable salts, solvates, crystals or prodrugs thereof, their preparation methods, pharmaceutical compositions containing these compounds, and use of these compounds or compositions for the treatment of bacterial infections.

### Background Art

The discovery and use of antibiotics, as one of the greatest medical achievements in the 20th century, saved countless lives. However, in the past few decades, the emergence of drug-resistant bacteria is seriously threatening people's lives.

β-Lactam antibiotics are one of the most commonly used antibiotics, and the drug resistance against them has gradually emerged. For most gram-negative bacteria, the resistance to β-lactam antibiotics is mainly driven by β-lactamase, which can hydrolyze β-lactam and cause inactivation of the antibiotics. There are 4 kinds of β-lactamases (MBLs), namely Types A, B, C and D. The expression of these β-lactamases is becoming a serious threat in relation to the drug resistance of bacteria.

Currently, the main monocyclic antibiotics on the market are aztreonam and carumonan. Although they are not easily hydrolyzed by MBLs, their weak activities against pseudomonas and acinetobacter also limit their clinical use. Therefore, looking for more active monocyclic antibiotics has become an urgent clinical need.

### Summary of the Invention

An object of the present invention is to provide a compound represented by general formula (I), or an isomer, pharmaceutically acceptable salt, solvate, crystal or prodrug thereof,

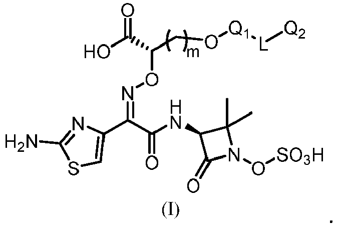

Another object of the present invention is to provide a method for preparing the compound of general formula (I) of the present invention, or an isomer, pharmaceutically acceptable salt, solvate, crystal or prodrug thereof.

Another object of the present invention is to provide a composition comprising the compound of general formula (I) of the present invention, or an isomer, pharmaceutically acceptable salt, solvate, crystal or prodrug thereof, and a pharmaceutically acceptable carrier, and a composition comprising the compound of general formula (I) of the present invention, or an isomer, pharmaceutically acceptable salt, solvate, crystals or prodrug thereof, and one or more other drugs.

Another object of the present invention is to provide a method for the treatment of a bacterial infection with a compound of the general formula (I) of the present invention, or an isomer, pharmaceutically acceptable salt, solvate, crystal or prodrug thereof, and use of a compound of the general formula (I) of the present invention, or an isomer, pharmaceutically acceptable salt, solvate, crystal or prodrug thereof, in the preparation of a medicament for the treatment of a bacterial infection.

In respect of the above-mentioned objects, the present invention provides the following technical solutions:
In a first aspect, the present invention provides a compound represented by general formula (I), or an isomer, pharmaceutically acceptable salt, solvate, crystal or prodrug thereof, 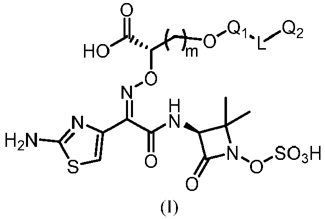 wherein
Q₁ is selected from the group consisting of aryl, aryl-fused-heterocyclyl, heteroaryl-fused-heterocyclyl, 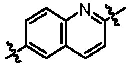 and 5-membered heteroaryl, which is optionally substituted by one or more R¹;
Q2 is absent or is selected from the group consisting of 4-6 membered heterocyclyl, aryl-fused-heterocyclyl, heteroaryl-fused-cycloalkyl, aryl-fused-heteroaryl, heteroaryl-fused-heterocyclyl, 5-membered heteroaryl and 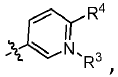 which is optionally substituted by one or more R², R³ is absent or is R², and R⁴ is selected from the group consisting of aminoalkylamido and hydroxyaminoalkylamido;
R¹ is selected from the group consisting of halogen, hydroxy, alkyl, haloalkyl, hydroxyalkyl, alkoxy, haloalkoxy, hydroxyalkoxy, nitro, carboxy, cyano, amino, monoalkylamino, alkylamido, alkylacyl, carbamoyl, alkylcarbamoyl, dialkylamino, alkenyl, alkynyl, cycloalkyl, heterocyclyl, aryl, heteroaryl and oxo;
R² is selected from the group consisting of halogen, hydroxy, alkyl, haloalkyl, hydroxyalkyl, aminoalkyl, alkoxy, haloalkoxy, hydroxyalkoxy, aminoalkoxy, nitro, carboxy, cyano, amino, monoalkylamino, alkylamido, aminoalkylamido, hydroxyaminoalkylamido, alkylacyl, carbamoyl, alkylcarbamoyl, dialkylamino, alkenyl, alkynyl, cycloalkyl, heterocyclyl, heterocyclylalkyl, aminoheterocyclyl, aminoalkylheterocyclyl, hydroxyalkylheterocyclyl, aryl, heteroaryl and oxo, which is optionally substituted by amino, aminoalkyl, alkylamino, halogen, hydroxy, alkyl, haloalkyl, hydroxyalkyl, alkoxy, haloalkoxy, hydroxyalkoxy, aminoalkoxy, nitro, carboxy or cyano;
L is absent or is selected from the group consisting of -C(O)NH-, -NHC(O)-, -SO₂NH-, -NHSO₂, -(CH₂)ₙ- and -C(O)-;
m is 1, 2, 3 or 4;
n is 1, 2, 3 or 4; and
when Q₁ is aryl, Q₂ is selected from the group consisting of aryl-fused-heterocyclyl, heteroaryl-fused-cycloalkyl, aryl-fused-heteroaryl and heteroaryl-fused-heterocyclyl; or
when Q₁ is aryl, Q₂ is 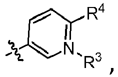 wherein R³ is absent or is R², and R⁴ is selected from the group consisting of aminoalkylamido and hydroxyaminoalkylamido; or
when Q₁ is aryl, Q₂ is 5-membered heteroaryl, and L is selected from the group consisting of -C(O)NH-, -NHC(O)-, -SO₂NH-, -NHSO₂-, -(CH₂)ₙ- and -C(O)-; or
when Q₁ is aryl, Q₂ is 5-membered heteroaryl, L is absent, and m is 2, 3 or 4; or
when Q₁ is selected from the group consisting of aryl-fused-heterocyclyl and heteroaryl-fused-heterocyclyl, Q₂ is 5-membered heteroaryl, and L is absent; or
when Q₁ is 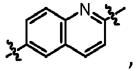 L is absent, and Q₂ is 4-6 membered heterocyclyl substituted by R²; or
when Q₁ is 5-membered heteroaryl, Q₂ is absent, and L is absent.

In some embodiments, in the compound of general formula (I) of the present invention, or an isomer, pharmaceutically acceptable salt, solvate, crystal or prodrug thereof, Q₁ is selected from the group consisting of 6-18 membered aryl, 9-20 membered aryl-fused-heterocyclyl, 9-20 membered heteroaryl-fused-heterocyclyl, 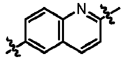 and 5-membered heteroaryl, which is optionally substituted by one or more R¹.

More preferably, Q₁ is selected from the group consisting of 6-12 membered aryl, 9-12 membered aryl-fused-heterocyclyl, 9-12 membered heteroaryl-fused-heterocyclyl, 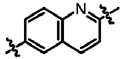 and 5-membered heteroaryl, which is optionally selected by one or more R¹.

Even more preferably, Q₁ is selected from the group consisting of phenyl, indolinyl, isoindolinyl, dihydroisobenzofuran, dihydrobenzopyranyl, dihydrobenzothienyl, benzoxazolinonyl, dihydrobenzopyrazolyl, dihydrobenzimidazolyl, dihydrobenzopyrazolyl, dihydrobenzoxazolyl, dihydrobenzothiazolyl, dihydrobenzoisoxazolyl, dihydrobenzoisothiazolyl, tetrahydroquinolinyl, tetrahydroisoquinolinyl, dihydroquinolinyl, dihydroisoquinolinyl, tetrahydroquinazolinyl, dihydroquinazolinyl, dihydrocinnolinyl, tetrahydrocinnolinyl, dihydroquinoxalinyl, tetrahydroquinoxalinyl, dihydrobenzoxazinyl, dihydrobenzothiazinyl, benzodioxinyl, benzomorpholinyl, benzoxathiolanyl, 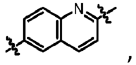 imidazolyl, pyrrolyl, furanyl, thienyl, pyrazolyl, oxazolyl, thiazolyl, isoxazolyl, isothiazolyl, oxadiazolyl and triazolyl, which is optionally substituted by one or more R¹.

In some preferred embodiments, in the compound of general formula (I) of the present invention, or an isomer, pharmaceutically acceptable salt, solvate, crystal or prodrug thereof, Q2 is absent or is selected from the group consisting of 4-6 membered heterocyclyl, 9-20 membered aryl-fused-cycloalkyl, 9-20 membered aryl-fused-heterocyclyl, 9-20 membered heteroaryl-fused-heterocyclyl, 5 membered heteroaryl and 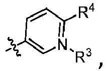 which is optionally substituted by one or more R², R³ is absent or is R², and R⁴ is selected from the group consisting of aminoC₁₋₁₂alkylamido and hydroxyaminoC₁₋₁₂alkylamido.

More preferably, Q₂ is absent or is selected from the group consisting of 4-6 membered heterocyclyl, 9-12 membered aryl-fused-cycloalkyl, 9-12 membered aryl-fused-heterocyclyl, 9-12 membered heteroaryl-fused-heterocyclyl, 5-membered heteroaryl and 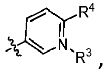 which is optionally substituted by one or more R², R³ is absent or is R², and R⁴ is selected from the group consisting of aminoC₁₋₆alkylamido and hydroxyaminoC₁₋₆alkylamido.

Even more preferably, Q₂ is absent or is selected from the group consisting of azetidinyl, tetrahydropyrrolyl, piperidinyl, piperazinyl, dihydrobenzofuranyl, dihydrobenzopyranyl, dihydrobenzothienyl, benzoxazolinonyl, dihydrobenzopyrazolyl, dihydrobenzimidazolyl, dihydrobenzopyrazolyl, dihydrobenzoxazolyl, dihydrobenzothiazolyl, dihydrobenzisoxazolyl, dihydrobenzisothiazolyl, tetrahydroquinolinyl, tetrahydroisoquinolinyl, dihydroquinolinyl, dihydroisoquinolinyl, tetrahydroquinazolinyl, dihydroquinazolinyl, dihydrocinnolinyl, tetrahydrocinnolinyl, dihydroquinoxalinyl, tetrahydroquinoxalinyl, dihydrobenzoxazinyl, dihydrobenzothiazinyl, benzodioxinyl, benzomorpholinyl, benzoxathiolanyl, tetrahydropyrrolopyrazolyl, tetrahydropiperidinopyrazolyl, dihydropiperidinopyrazolyl, pyrazolodihydropyrazolyl, imidazolyl, pyrrolyl, furanyl, thienyl, pyrazolyl, oxazolyl, thiazolyl, isoxazolyl, isothiazolyl, oxadiazolyl, triazolyl, indolyl, isoindolyl, benzopyrazolyl, benzimidazolyl, benzofuranyl, benzopyranyl, benzothienyl, benzoxazolyl, benzothiazolyl, benzisoxazolyl, benzisothiazolyl, quinolinyl, isoquinolinyl, quinazolinyl, dihydroquinazolinyl,

cinnolinyl, quinoxalinyl, benzoxazinyl, benzothiazinyl and 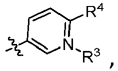 which is optionally substituted by one or more R², R³ is absent or is R², and R⁴ is selected from the group consisting of

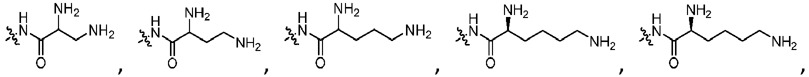

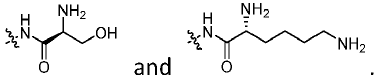

In some preferred embodiments, in the compound of general formula (I) of the present invention, or an isomer, pharmaceutically acceptable salt, solvate, crystal or prodrug thereof, R¹ is selected from the group consisting of halogen, hydroxy, C₁₋₆alkyl, haloC₁₋₆alkyl, hydroxyC₁₋₆alkyl, C₁₋₆alkoxy, haloC₁₋₆alkoxy, hydroxyC₁₋₆alkoxy, nitro, carboxy, cyano, amino, monoC₁₋₆alkylamino, C₁₋₆alkylamido, C₁₋₆alkylacyl, carbamoyl, C₁₋₆alkylcarbamoyl, diC₁₋₆alkylamino, C₂₋₁₀alkenyl, C₂₋₁₀alkynyl, C₃₋₁₂cycloalkyl, 3-12 membered heterocyclyl, 6-12 membered aryl, 5-12 membered heteroaryl and oxo.

More preferably, R¹ is selected from the group consisting of halogen, hydroxy, C₁₋₃alkyl, haloC₁₋₃alkyl, hydroxyC₁₋₃alkyl, C₁₋₃alkoxy, haloC₁₋₃alkoxy, hydroxyC₁₋₃alkoxy, nitro, carboxy, cyano, amino, monoC₁₋₃alkylamino, C₁₋₃alkylamido, C₁₋₃alkylacyl, carbamoyl, C₁₋₃alkylcarbamoyl, diC₁₋₃alkylamino, C₂₋₆alkenyl, C₂₋₆alkynyl, C₃₋₈cycloalkyl, 3-8 membered heterocyclyl, 6-10 membered aryl, 5-10 membered heteroaryl and oxo.

Even more preferably, R¹ is selected from the group consisting of hydrogen, fluorine, chlorine, bromine, iodine, hydroxy, methyl, ethyl, propyl, isopropyl, cyclopropyl, cyclobutyl, cyclopentyl, cyclohexyl, trifluoromethyl, trifluoroethyl, hydroxymethyl, hydroxyethyl, hydroxypropyl, 2-hydroxypropyl, methoxy, ethoxy, propoxy, isopropoxy, trifluoromethoxy, hydroxylmethoxy, hydroxyethoxy, hydroxypropoxy, nitro, carboxy, cyano, amino, methylamino, dimethylamino, ethylamino, diethylamino, methylethylamino, methylamido, ethylamido, vinylamido, methylacyl, ethylacyl, vinylacyl, carbamoyl, methylcarbamoyl, ethylcarbamoyl, vinyl, ethynyl, C₃₋₆cycloalkyl, 3-6 membered heterocyclyl, 6-8 membered aryl, 5-8 membered heteroaryl and oxo.

In some preferred embodiments, in the compound of general formula (I) of the present invention, or an isomer, pharmaceutically acceptable salt, solvate, crystal or prodrug thereof, R² is selected from the group consisting of halogen, hydroxy, C₁₋₆alkyl, haloC₁₋₆alkyl, hydroxyC₁₋₆alkyl, aminoC₁₋₆alkyl, C₁₋₆alkoxy, haloC₁₋₆alkoxy, hydroxyC₁₋₆alkoxy, aminoC₁₋₆alkoxy, nitro, carboxy, cyano, amino, monoC₁₋₆alkylamino, C₁₋₆alkylamido, aminoC₁₋₁₂alkylamido, hydroxyaminoC₁₋₁₂alkylamido, amino 3-6 membered heterocyclyl, C₁₋₆alkylacyl, carbamoyl, C₁₋₆alkylcarbamoyl, diC₁₋₆alkylamino, C₂₋₁₀alkenyl, C₂₋₁₀alkynyl, C₃₋₁₂cycloalkyl, 3-12 membered heterocyclyl, 3-12 membered heterocyclyl C₁₋₆alkyl, aminoC₁₋₆alkyl 3-12 membered heterocyclyl, hydroxyC₁₋₆alkyl 3-12 membered heterocyclyl, 6-12 membered aryl, 5-12 membered heteroaryl and oxo, which is optionally substituted by amino, aminoC₁₋₆alkyl, C₁₋₆alkylamino, halogen, hydroxy, C₁₋₆alkyl, haloC₁₋₆alkyl, hydroxyC₁₋₆alkyl, C₁₋₆alkoxy, haloC₁₋₆alkoxy, hydroxyC₁₋₆alkoxy, aminoC₁₋₆alkoxy, nitro, carboxy or cyano.

More preferably, R² is selected from the group consisting of halogen, hydroxy, C₁₋₃alkyl, haloC₁₋₃alkyl, hydroxyC₁₋₃alkyl, aminoC₁₋₆alkyl, C₁₋₃alkoxy, haloC₁₋₃alkoxy, hydroxyC₁₋₃alkoxy, aminoC₁₋₃alkoxy, nitro, carboxy, cyano, amino, monoC₁₋₃alkylamino, C₁₋₃alkylamido, aminoC₁₋₆alkylamido, hydroxyaminoC₁₋₆alkylamido, C₁₋₃alkylacyl, carbamoyl, C₁₋₃alkylcarbamoyl, diC₁₋₃alkylamino, C₂₋₆alkenyl, C₂₋₆alkynyl, C₃₋₈cycloalkyl, 3-8 membered heterocyclyl, 3-8 membered heterocyclyl C₁₋₃alkyl, amino 3-6 membered heterocyclyl, aminoC₁₋₃alkyl 3-8 membered heterocyclyl, hydroxyC₁₋₃alkyl 3-8 membered heterocyclyl, 6-10 membered aryl, 5-10 membered heteroaryl and oxo, which is optionally substituted by amino, aminoC₁₋₆alkyl, C₁₋₆alkylamino, halogen, hydroxy, C₁₋₆alkyl, haloC₁₋₆alkyl, hydroxyC₁₋₆alkyl, C₁₋₆alkoxy, haloC₁₋₆alkoxy, hydroxyC₁₋₆alkoxy, aminoC₁₋₆alkoxy, nitro, carboxy or cyano.

Even more preferably, R² is selected from the group consisting of hydrogen, fluorine, chlorine, bromine, iodine, hydroxy, methyl, ethyl, propyl, isopropyl, cyclopropyl, cyclobutyl, cyclopentyl, cyclohexyl, trifluoromethyl, trifluoroethyl, hydroxymethyl, hydroxyethyl, hydroxypropyl, 2-hydroxypropyl, aminomethyl, aminoethyl, aminopropyl, aminobutyl, methoxy, ethoxy, propoxy, isopropoxy, trifluoromethoxy, hydroxymethoxy, hydroxyethoxy, hydroxypropoxy, aminomethoxy, aminoethoxy, aminopropoxy, nitro, carboxy, cyano, amino, methylamino, dimethylamino, ethylamino, diethylamino, methylethylamino, methylamido, ethylamido, vinylamido, aminoC₁₋₆alkylamido, hydroxylaminoC₁₋₆alkylamido, methylacyl, ethylacyl, vinylacyl, carbamoyl, methylcarbamoyl, ethylcarbamoyl, vinyl, ethynyl, C₃₋₆cycloalkyl, 3-6 member heterocyclyl, 3-6 membered heterocyclyl C₁₋₃alkyl, amino 3-6 membered heterocyclyl, aminoC₁₋₃alkyl 3-6 membered heterocyclyl, hydroxyC₁₋₃alkyl 3-6 member heterocyclyl, 6-8 membered aryl, 5-8 membered heteroaryl and oxo, which is optionally substituted by amino, aminoC₁₋₆alkyl, C₁₋₆alkylamino, halogen, hydroxy, C₁₋₆alkyl, haloC₁₋₆alkyl, hydroxyC₁₋₆alkyl, C₁₋₆alkoxy, haloC₁₋₆alkoxy, hydroxyC₁₋₆alkoxy, aminoC₁₋₆alkoxy, nitro, carboxyl or cyano.

In some specific embodiments, R² is selected from the group consisting of hydrogen, fluorine, chlorine, bromine, iodine, hydroxy, methyl, ethyl, propyl, isopropyl, cyclopropyl, cyclobutyl, cyclopentyl, cyclohexyl, trifluoromethyl, trifluoroethyl, hydroxymethyl, hydroxyethyl, hydroxypropyl, 2-hydroxypropyl, aminomethyl, aminoethyl, aminopropyl, aminobutyl, methoxy, ethoxy, propoxy, isopropoxy, trifluoromethoxy, hydroxymethoxy, hydroxyethoxy, hydroxypropoxy, aminomethoxy, aminoethoxy, aminopropoxy, nitro, carboxy, cyano, amino, methylamino, dimethylamino, ethylamino, diethylamino, methylethylamino, methylamido, ethylamido, vinylamido, aminoC₁₋₆alkylamido, hydroxyaminoC₁₋₆alkylamido, methylacyl, ethylacyl, vinylacyl, carbamoyl, methylcarbamoyl, ethylcarbamoyl, vinyl, ethynyl, C₃₋₆cycloalkyl, 3-6 membered azacycloalkyl, 3-6 membered azacycloalkyl C₁₋₃alkyl, amino 3-6 membered azacycloalkyl, aminoC₁₋₃alkyl 3-6 membered azacycloalkyl, hydroxyC₁₋₃alkyl 3-6 membered azacycloalkyl, 6-8 membered aryl, 5-8 membered heteroaryl and oxo, which is optionally substituted by amino, aminoC₁₋₃alkyl , C₁₋₃alkylamino, halogen, hydroxy, C₁₋₃alkyl, haloC₁₋₃alkyl, hydroxyC₁₋₃alkyl, C₁₋₃alkoxy, haloC₁₋₃alkoxy, hydroxyC₁₋₃alkoxy, aminoC₁₋₃ alkoxy, nitro, carboxy or cyano.

In some embodiments, R² is selected from the group consisting of methyl, ethyl, propyl, isopropyl, azetidinyl, azetidinylmethyl, azetidinylethyl, azetidinylpropyl, aminomethyl, aminoethyl, aminopropyl, aminomethoxy, aminoethoxy, aminopropoxy, aminomethylamido, aminoethylamido, aminopropylamido, aminobutylamido, aminopentylamido, hydroxymethylamido, hydroxyethylamido, hydroxypropylamido, aminoaziridinyl, aminoazetidinyl and aminopyrrolidinyl, which is optionally substituted by amino, aminoC₁₋₃alkyl, C₁₋₃alkylamino, halogen, hydroxy, C₁₋₃alkyl, haloC₁₋₃alkyl, hydroxyC₁₋₃alkyl, C₁₋₃alkoxy, haloC₁₋₃alkoxy, hydroxyC₁₋₃alkoxy, aminoC₁₋₃alkoxy, nitro, carboxy or cyano.

In some preferred embodiments, in the compound of general formula (I) of the present invention, or an isomer, pharmaceutically acceptable salt, solvate, crystal or prodrug thereof, Q₁ is 6-18 membered aryl, which is optionally substituted by one or more R¹; and Q₂ is selected from the group consisting of 9-20 membered aryl-fused-heterocyclyl, 9-20 membered heteroaryl-fused-cycloalkyl, 9-20 membered aryl-fused-heteroaryl, 9-20 membered heteroaryl-fused-heterocyclyl, which is optionally substituted by one or more R².

More preferably, Q₁ is 6-12 membered aryl, which is optionally substituted by one or more R¹; and Q₂ is selected from the group consisting of 9-12 membered aryl-fused-heterocyclyl, 9-12 membered heteroaryl-fused-cycloalkyl, 9-12 membered aryl-fused-heteroaryl, and 9-12 membered heteroaryl-fused-heterocyclyl, which is optionally substituted by one or more R².

Even more preferably, Q₁ is phenyl, which is optionally substituted by one or more R¹; and Q₂ is selected from the group consisting of dihydrobenzofuranyl, dihydrobenzopyranyl, dihydrobenzothienyl, benzoxazolinonyl, dihydrobenzopyrazolyl, dihydrobenzimidazolyl, dihydrobenzopyrazolyl, dihydrobenzoxazolyl, dihydrobenzothiazolyl, dihydrobenzisoxazolyl, dihydrobenzisothiazolyl, tetrahydroquinolinyl, tetrahydroisoquinolinyl, dihydroquinolinyl, dihydroisoquinolinyl, tetrahydroquinazolinyl, dihydroquinazolinyl, dihydrocinnolinyl, tetrahydrocinnolinyl, dihydroquinoxalinyl, tetrahydroquinoxalinyl, dihydrobenzoxazinyl, dihydrobenzothiazinyl, benzodioxinyl, benzomorpholinyl, benzoxathiolanyl, tetrahydropyrrolopyrazolyl, tetrahydropiperidinopyrazolyl, dihydropiperidinopyrazolyl, pyrazolodihydropyrazolyl, indolyl, isoindolyl, benzopyrazolyl, benzimidazolyl, benzofuranyl, benzopyranyl, benzothienyl, benzoxazolyl, benzothiazolyl, benzisoxazolyl, benzisothiazolyl, quinolinyl, isoquinolinyl, quinazolinyl, dihydroquinazolinyl, cinnolinyl, quinoxalinyl, benzoxazinyl, benzothiazinyl, which is optionally substituted by one or more R².

In some preferred embodiments, the compound of the present invention is a compound of general formula (Ia), or an isomer, pharmaceutically acceptable salt, solvate, crystal or prodrug thereof, 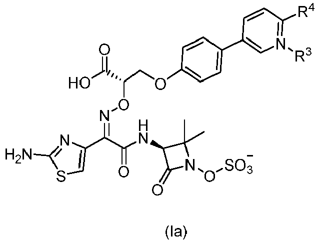 wherein
R³ is absent or is R², wherein R² has the definition described in the above general formula (I); and
R⁴ is selected from the group consisting of aminoalkylamido and hydroxyaminoalkylamido.

In some preferred embodiments, in the compound of general formula (I) or (Ia) of the present invention, or an isomer, pharmaceutically acceptable salt, solvate, crystal or prodrug thereof, R⁴ is selected from the group consisting of aminoC₁₋₁₂alkylamido and hydroxyaminoC₁₋₁₂alkylamido.

More preferably, R⁴ is selected from the group consisting of aminoC₁₋₆alkylamido and hydroxyaminoC₁₋₆alkylamido.

Even more preferably, R⁴ is selected from the group consisting of

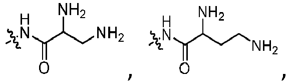

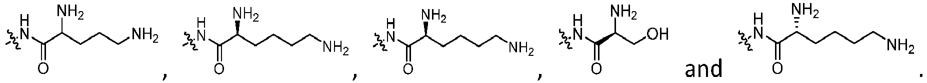

In some preferred embodiments, in the compound of general formula (I) of the present invention, or an isomer, pharmaceutically acceptable salt, solvate, crystal or prodrug thereof, Q₁ is 6-18 membered aryl, which is optionally substituted by one or more R¹; Q₂ is 5-membered heteroaryl, which is optionally substituted by one or more R²; and L is selected from the group consisting of -C(O)NH-, -NHC(O)-, -SO₂NH-, -NHSO₂-, -(CH₂)ₙ- and -C(O)-.

More preferably, Q₁ is 6-12 membered aryl, which is optionally substituted by one or more R¹; Q₂ is 5-membered heteroaryl, which is optionally substituted by one or more R²; and L is selected from the group consisting of -C(O)NH-, -NHC(O)-, -SO₂NH-, -NHSO₂-, -(CH₂)ₙ- and -C(O)-.

Even more preferably, Q₁ is phenyl, which is optionally substituted by one or more R¹; Q₂ is selected from the group consisting of imidazolyl, pyrrolyl, furanyl, thienyl, pyrazolyl, oxazolyl, thiazolyl, isoxazolyl, isothiazolyl, oxadiazolyl and triazolyl, which is optionally substituted by one or more R²; and L is selected from the group consisting of -C(O)NH-, -NHC(O)-, -SO₂NH-, -NHSO₂-, -(CH₂)ₙ- and -C(O)-.

In some preferred embodiments, the compound of the present invention is a compound of general formula (Ib) or an isomer, pharmaceutically acceptable salt, solvate, crystal or prodrug thereof, 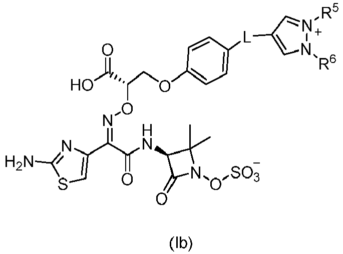 wherein L is selected from the group consisting of -C(O)NH-, -NHC(O)-, -SO₂NH-, -NHSO₂, -(CH₂)ₙ- and -C(O)-, R⁵ and R⁶ each independently have the same definition as described above for R² in formula (I).

In some preferred embodiments, in the compound of general formula (Ib) of the present invention, or an isomer, pharmaceutically acceptable salt, solvate, crystal or prodrug thereof, R⁵ is selected from the group consisting of methyl, ethyl, propyl, cyclopropyl, 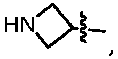 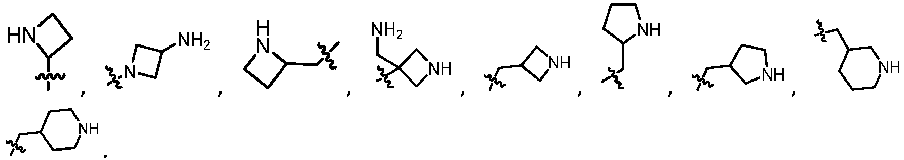 and

In some preferred embodiments, in the compound of general formula (Ib) of the present invention, or an isomer, pharmaceutically acceptable salt, solvate, crystal or prodrug thereof, R⁶ is selected from the group consisting of

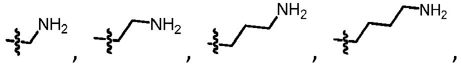

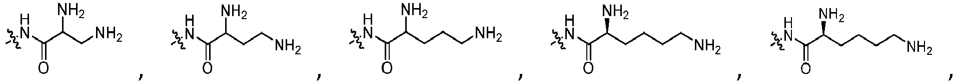

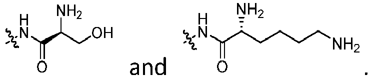

In some preferred embodiments, in the compound of general formula (I) of the present invention, or an isomer, pharmaceutically acceptable salt, solvate, crystal or prodrug thereof, Q₁ is 6-18 membered aryl, which is optionally substituted by one or more R¹; Q₂ is 5-membered heteroaryl, which is optionally substituted by one or more R²; L is absent; and m is 2, 3 or 4.

More preferably, Q₁ is 6-12 membered aryl, which is optionally substituted by one or more R¹; Q2 is a 5-membered heteroaryl, which is optionally substituted by one or more R²; L is absent; and m is 2, 3 or 4.

Even more preferably, Q₁ is phenyl, which is optionally substituted by one or more R¹; Q₂ is selected from the group consisting of imidazolyl, pyrrolyl, furanyl, thienyl, pyrazolyl, oxazolyl, thiazolyl, isoxazolyl, isothiazolyl, oxadiazolyl and triazolyl, which is optionally substituted by one or more R²; L is absent; and m is 2, 3 or 4.

In some preferred embodiments, in the compound of general formula (I) of the present invention, or an isomer, pharmaceutically acceptable salt, solvate, crystal or prodrug thereof, Q₁ is 9-20 membered aryl-fused-heterocyclyl, or 9-20 membered heteroaryl-fused-heterocyclyl, which is optionally substituted by one or more R¹; Q₂ is 5-membered heteroaryl, which is optionally substituted by one or more R²; and L is absent.

More preferably, Q₁ is selected from the group consisting of 9-12 membered aryl-fused-heterocyclyl and 9-12 membered heteroaryl-fused-heterocyclyl, which is optionally substituted by one or more R¹; Q₂ is 5-membered heteroaryl, which is optionally substituted by one or more R²; and L is absent.

Even more preferably, Q₁ is selected from the group consisting of indolinyl, isoindolinyl, dihydroisobenzofuran, dihydrobenzopyranyl, dihydrobenzothienyl, benzoxazolinonyl, dihydrobenzopyrazolyl, dihydrobenzimidazolyl, dihydrobenzopyrazolyl, dihydrobenzoxazolyl, dihydrobenzothiazolyl, dihydrobenzoisoxazolyl, dihydrobenzoisothiazolyl, tetrahydroquinolinyl, tetrahydroisoquinolinyl, dihydroquinolinyl, dihydroisoquinolinyl, tetrahydroquinazolinyl, dihydroquinazolinyl, dihydrocinnolinyl, tetrahydrocinnolinyl, dihydroquinoxalinyl, tetrahydroquinoxalinyl, dihydrobenzoxazinyl, dihydrobenzothiazinyl, benzodioxinyl, benzomorpholinyl and benzoxathiolanyl; Q₂ is selected from the group consisting of imidazolyl, pyrrolyl, furanyl, thienyl, pyrazolyl, oxazolyl, thiazolyl, isoxazolyl, isothiazolyl, oxadiazolyl and triazolyl, which is optionally substituted by one or more R²; and L is absent.

In some preferred embodiments, in the compound of general formula (I) of the present invention, or an isomer, pharmaceutically acceptable salt, solvate, crystal or prodrug thereof, Q₁ is 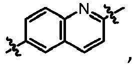 which is optionally substituted by one or more R¹; L is absent, Q₂ is selected from the group consisting of azetidinyl, tetrahydropyrrolyl, piperidinyl and piperazinyl, which is substituted by one or more R²; and L is absent.

In some preferred embodiments, in the compound of general formula (I) of the present invention, or an isomer, pharmaceutically acceptable salt, solvate, crystal or prodrug thereof, Q₁ is selected from the group consisting of imidazolyl, pyrrolyl, furanyl, thienyl, pyrazolyl, oxazolyl, thiazolyl, isoxazolyl, isothiazolyl, oxadiazolyl and triazolyl, which is optionally selected by one or more R¹;
Q₂ is absent; and
L is absent.

In some embodiments, the present invention provides inorganic salts and organic salts of the compound of general formula (I) of the present invention, or an isomer, a pharmaceutically acceptable salt, a solvates, a crystal or a prodrug thereof. Preferably, a pharmaceutically acceptable salt is hydrochloride, hydrobromide, phosphate, sulfamate, nitrate, p-toluenesulfonate, benzenesulfonate, p-aminobenzenesulfonate, methanesulfonate, sulfate, acetate, oxalate, phenylacetate, propionate, malonate, trifluoroacetate, succinate, glycolate, stearate, ascorbate, pamoate, hydroxymaleate, glutamate, benzoate, salicylate, 2-acetoxybenzoate, fumarate, ethanedisulfonate, oxalate, isethionate, citrate, D-gluconate, lactate, L-malate, succinate, L-tartrate, fumarate, α-ketoglutarate, hippurate, maleate, or D-tartrate, preferably hydrochloride, hydrobromide, phosphate, sulfamate, nitrate, p-toluenesulfonate, benzenesulfonate, p-aminobenzenesulfonate, methanesulfonate, sulfate, acetate, oxalate, phenylacetate, propionate, malonate, D-tartrate or trifluoroacetate. More preferably, the pharmaceutically acceptable salt is trifluoroacetate. In some specific embodiments, the invention provides a trifluoroacetate salt of a compound of the present invention.

The present invention provides the following specific compounds, or isomers, pharmaceutically acceptable salts, solvates, crystals or prodrugs thereof:

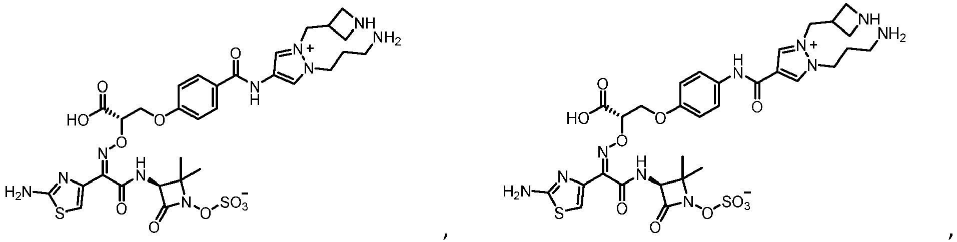

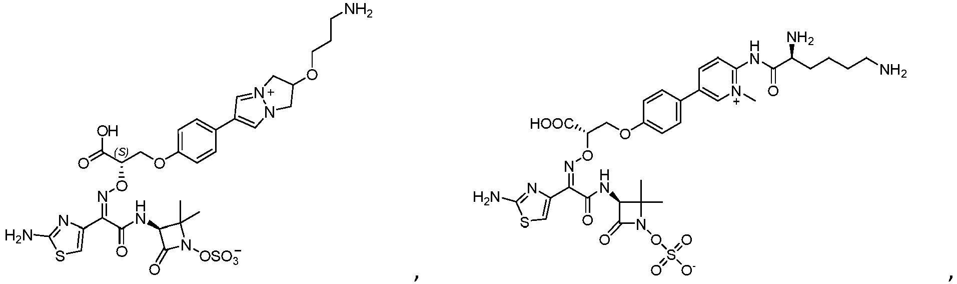

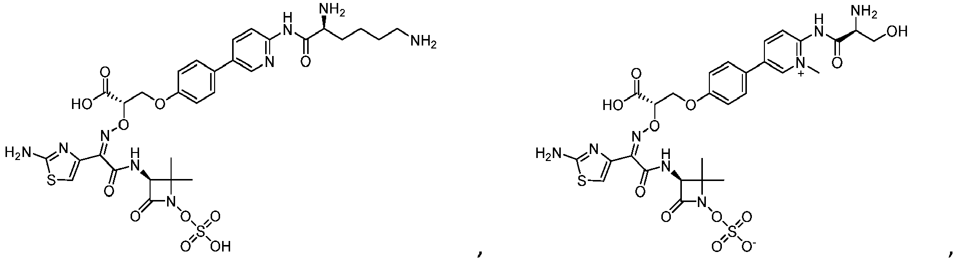

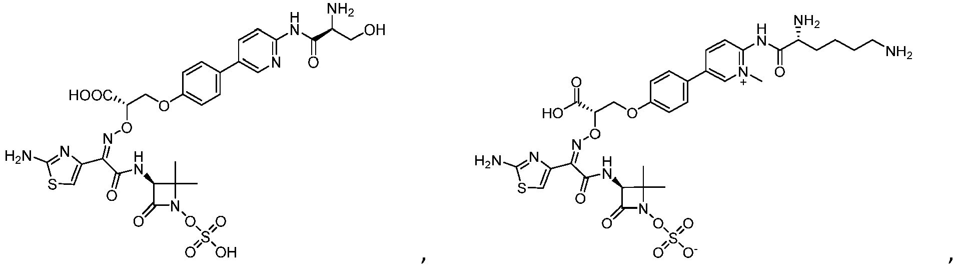

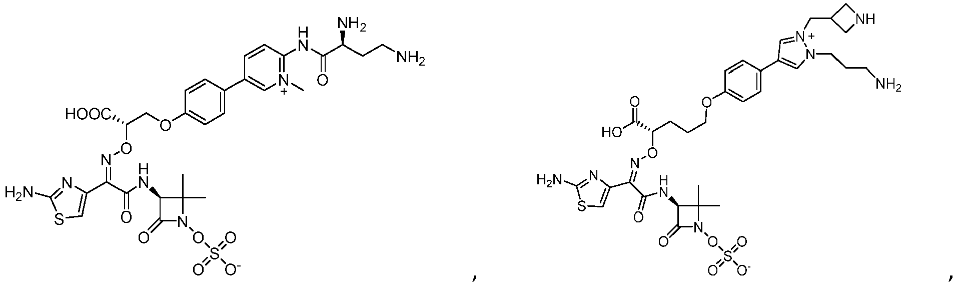

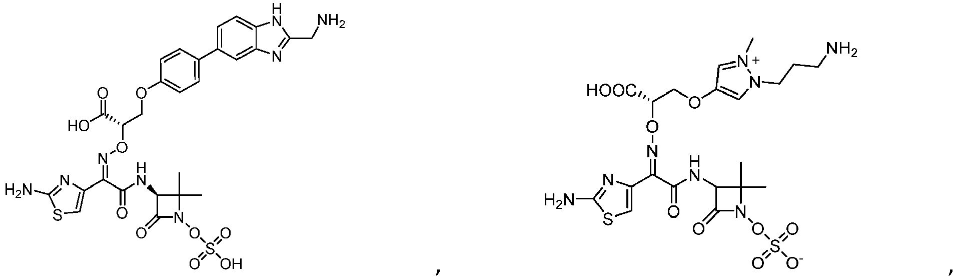

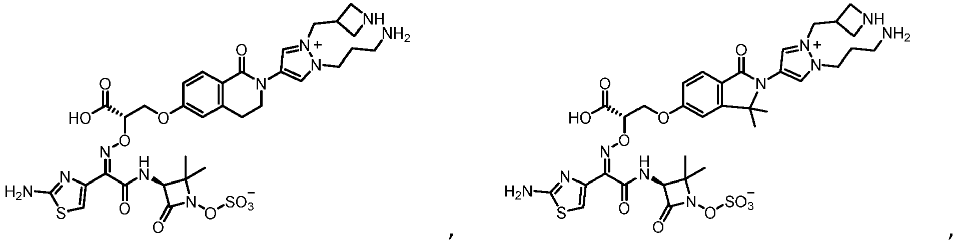

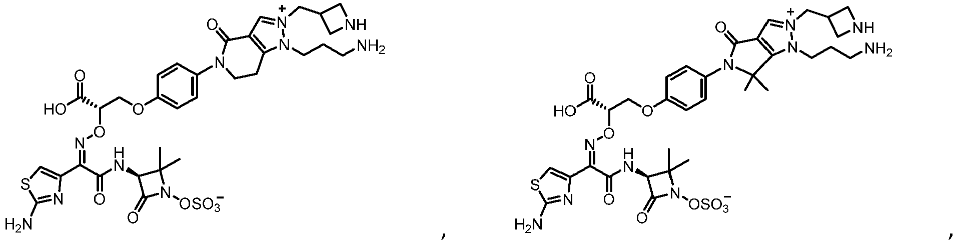

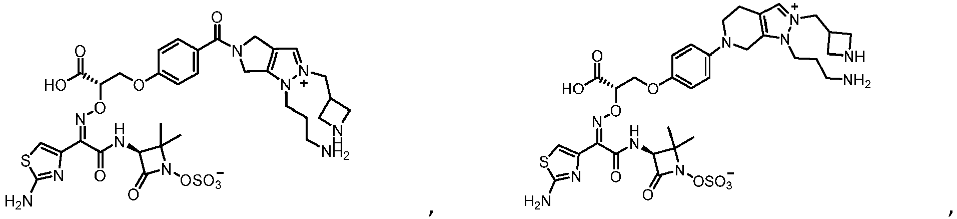

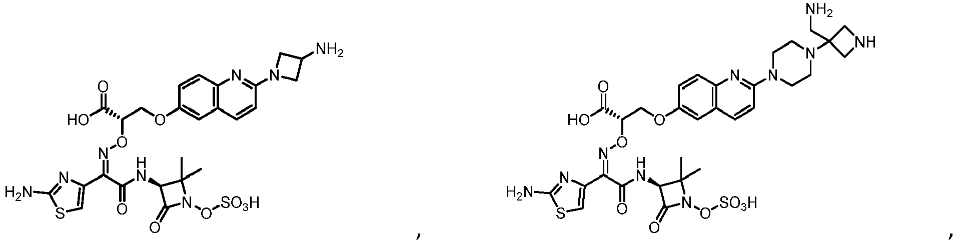

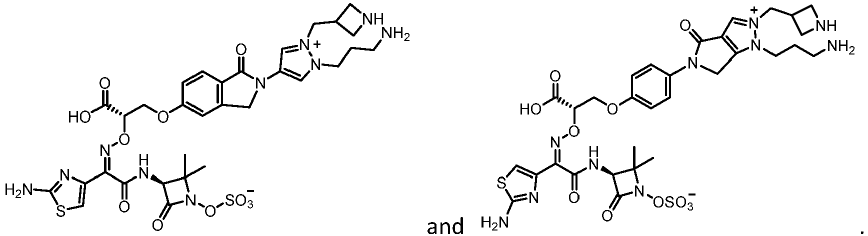

In another aspect, the present invention provides a method for preparing the compound of the general formula of the present invention. For example, the method for preparing the compound of formula (I) of the present invention includes but is not limited to the following steps: 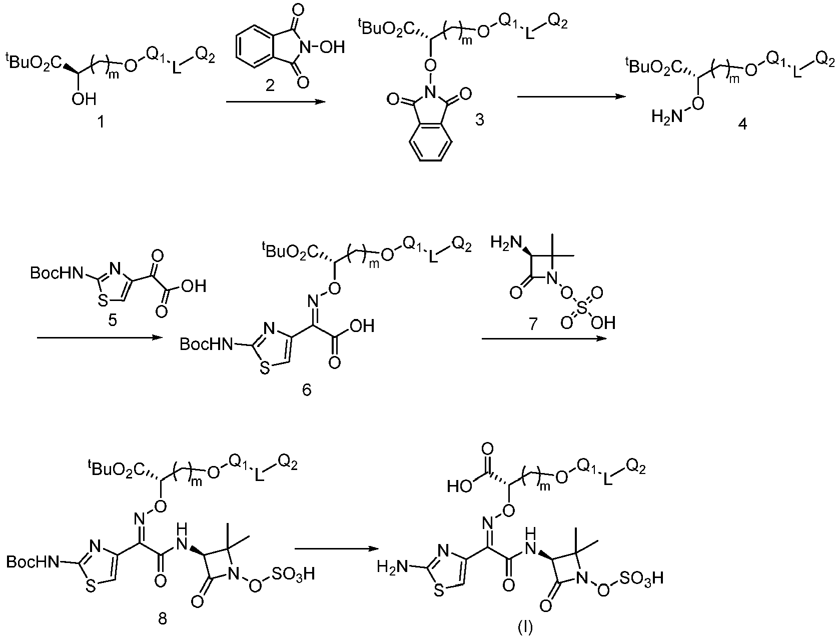
1) a compound of formula 1 is reacted with a compound of formula 2 to produce a compound of formula 3;
2) the compound of formula 3 is reacted to produce a compound of formula 4;
3) the compound of formula 4 is reacted with a compound of formula 5 to produce a compound of formula 6;
4) the compound of formula 6 is reacted with a compound of formula 7 to produce a compound of formula 8;
5) the amino protecting group is removed from the compound of formula 8 to produce the compound of formula (I),
wherein Q₁, Q₃, L and m have the same definitions as described for the general formula (I), and the compounds of formula 1, formula 2, formula 5 and formula 7 are commercially available compounds or can be synthesized following other technical means commonly used by a person skilled in the art.

In a third aspect, the present invention provides a pharmaceutical composition comprising a compound of the present invention, or an isomer, pharmaceutically acceptable salt, solvate, crystal or prodrug thereof.

In some embodiments, the present invention provides a pharmaceutical composition comprising a compound of the present invention, or an isomer, pharmaceutically acceptable salt, solvate, crystal or prodrug thereof, as well as other compounds including but not limited to monocyclic lactam antibiotics, such as β-lactam antibiotics, and additional antibiotics and/or additional β-lactamase inhibitors, and any other compounds sensitive to serine β-lactamase which are used in combination. In some embodiments, the present invention provides a pharmaceutical composition comprising a compound of the present invention, or an isomer, pharmaceutically acceptable salt, solvate, crystal or prodrug thereof, in combination with one or more of the following: penicillin, methicillin, oxacillin, nafcillin, cloxacillin, dicloxacillin, flucloxacillin, temocillin, amoxicillin, ampicillin, amoxicillin, azlocillin, carbenicillin, ticarcillin, mezlocillin, piperacillin, cephalexin, cephalothin, CXA-101, cefazolin, cefaclor, cefuroxim, cefamandole, cefotetan, cefoxitin, ceftriaxone, cefotaxime, cefpodoxime, cefixime, ceftazidime, ceftobiprole, cefepime, cefpirome, ceftaroline, imipenem, meropenem, ertapenem, faropenem, sulopenem, doripenem, PZ-601 (Protez Pharmaceuticals), ME1036 (Forest Labs), BAL30072, MC-1, tomopenem, tebipenem, aztreonam, tigemonam, nocardicin A, tabtoxinine, sulbactam, tazobactam, avibactam, amoxicillin, LK-157, LK-176, SA-1-204, SA-2-13, BLI-489 (Pfizer/Wyeth), BAL0029880 or MK7655, aminoglycosides, spectinomycins, macrolides, ketolides, streptogramins, oxazolidinones, tetracyclines, fluoroquinolones, coumarin antibiotics, glycopeptides, lipoglycopeptides, nitiomidazoles, ansamycins, phenylpropanols, mupirocyn, fosfomycin, tobramycin, linezolid, daptomycin, vancomycin or antimicrobial agents. In some embodiments, the present invention provides a pharmaceutical composition comprising a compound of the present invention, or an isomer, pharmaceutically acceptable salt, solvate, crystal or prodrug thereof, in combination with meropenem, aztreonam or ceftazidime. In some embodiments, the present invention provides a pharmaceutical composition comprising a compound of the present invention, or an isomer, pharmaceutically acceptable salt, solvate, crystal or prodrug thereof, in combination with avibactam, tazobactam, sulbactam and clavulanic acid.

In some embodiments, the present invention provides a compound of the present invention, or an isomer, pharmaceutically acceptable salt, solvate, crystal or prodrug thereof, and a pharmaceutical composition comprising a compound of the present invention, or an isomer, pharmaceutically acceptable salt, solvate, crystal or prodrug thereof, for used to treat a bacterial infection.

In some embodiments, the present invention provides a pharmaceutical composition comprising a compound of the present invention, or an isomer, pharmaceutically acceptable salt, solvate, crystal or prodrug thereof, and a pharmaceutically acceptable carrier.

The compound of the present invention, or an isomer, pharmaceutically acceptable salt, solvate, crystal or prodrug thereof, can be mixed with a pharmaceutically acceptable carrier, diluent or excipient to prepare a pharmaceutical formulation suitable for oral or parenteral administration. Administration methods include, but are not limited to, intradermal, intramuscular, intraperitoneal, intravenous, subcutaneous, intranasal and oral routes. The formulation can be administered by any route, for example, by infusion or bolus injection, by absorption through epithelium or skin mucosa (e.g., oral mucosa or rectum, etc.). The administration can be systemic or topical. Examples of the formulation for oral administration include solid or liquid dosage forms, specifically, tablets, pills, granules, powders, capsules, syrups, emulsions, suspensions, and the like. The formulation can be prepared by a method known in the art, and may contain a carrier, a diluent or an excipient conventionally used in the field of pharmaceutical formulation.

In a fourth aspect, the present invention provides a method for treating a bacterial infection with a compound represented by the general formula (I) of the present invention, or an isomer, pharmaceutically acceptable salt, solvate, crystal or prodrug thereof, or a pharmaceutical composition containing the same, and use thereof in the manufacture of a medicament for treating a bacterial infection.

In some preferred embodiments, the bacterial infection of the present invention is an infection caused by a gram-negative bacterium, also known as "gram-negative infection", wherein the gram-negative bacterium may be selected from the group consisting of the following genera: Citrobacter, Enterobacter, Escherichia, Klebsiella, Morganella, Proteus, Salmonella, Serratia, Pseudomonas, Acinetobacter, Bacteroides, Burkholderia, Campylobacter, Neisseria and Stenotrophomonas. In particular, infection caused by Citrobacter, Enterobacter, Escherichia, Klebsiella, Morganella, Proteus, Salmonella, Serratia, Pseudomonas or Acinetobacter can be treated. For the treatment, specific bacterial species include Citrobacter freundii, Citrobacter koseri, Enterobacter cloacae, Enterobacter faecalis, Enterobacter faecium, Escherichia coli, Klebsiella pneumoniae, Klebsiella oxytoca, Morganella morganii, Proteus mirabilis, Salmonella species, Serratia marcescens, Pseudomonas aeruginosa, Acinetobacter baumanii, Bacteroides bivius, Bacteroides fragilis, Burkholderia cepacia, Campylobacter jejuni, Neisseria gonorrhoeae and Stenotrophomonas maltophilia. In other preferred embodiments, the bacterial infection of the present invention is an infection caused by a drug resistant microorganism (including multi-drug resistant microorganism). In other preferred embodiments, the bacterial infection of the present invention is an infection caused by a multi-drug resistant microorganism. In other preferred embodiments, the gram-negative infection of the present invention is an infection resistant to one or more antibiotics. In other preferred embodiments, the gram-negative infection of the present invention is an infection resistant to multiple drugs.

In some preferred embodiments, the compound of the present invention can be used to treat infections caused by the following bacteria: Enterobacteriaceae, including Salmonella, Escherichia coli, Klebsiella pneumoniae, Proteus, Enterobacter, Serratia, Citrobacter, including pathogens, such as KPC-producing Klebsiella pneumonia which is less sensitive to previous monocyclic lactam antibiotics such as aztreonam, as well as nonfermentating bacilli, including Pseudomonas aeruginosa, Acinetobacter, Burkholderia, Moraxella and Stenotrophomonas.

In some preferred embodiments, the present invention provides a method for treating a bacterial infection with a compound represented by the general formula (I) of the present invention, or an isomer, pharmaceutically acceptable salt, solvate, crystal or prodrug thereof, or a pharmaceutical composition comprising the same, and use thereof in the manufacture of a medicament for treating a bacterial infection, wherein the bacterial infection includes, but is not limited to, gynecological infection, respiratory tract infection (RTI), urinary tract infection (UTI), complicated urinary tract infection (including pyelonephritis), sexually transmitted disease, acute exacerbation of chronic bronchitis (ACEB), acute otitis media, acute sinusitis, infection caused by drug resistant bacterium, catheter-associated septicemia, chancroid, chlamydia, pneumonia, prostatitis, community-acquired pneumonia (CAP), complicated skin and skin structure infection, non-complicated skin and skin structure infection, skin and soft tissue infection, endocarditis, febrile neutropenia, gonococcal meningitis, gonococcal urethritis, hospital-acquired pneumonia (HAP), ventilator-associated pneumonia (VAP), osteomyelitis, primary or secondary blood infection (septicemia), syphilis, intra-abdominal infection, complicated intra-abdominal infection, postoperative infection, etc. In some preferred embodiments, the present invention provides a method for treating a bacterial infection with a compound represented by the general formula (I) of the present invention, or an isomer, pharmaceutically acceptable salt, solvate, crystal or prodrug thereof, or a pharmaceutical composition comprising the same, and use thereof in the manufacture of a medicament for treating a bacterial infection, wherein the bacterial infection includes, but is not limited to, community-acquired pneumonia (CAP), hospital-acquired pneumonia (HAP), ventilator-related Pneumonia (VAP), primary or secondary blood infection (septicemia), complicated urinary tract infections (including pyelonephritis), complicated intra-abdominal infection, postoperative infection, etc.

In one embodiment, the present invention provides a method for treating one or more of the infections listed above, comprising administering to a subject suffered from a bacterial infection an effective amount of a compound of formula (I), or a pharmaceutically acceptable salt thereof, in combination with an additional antibiotic. In one aspect of this embodiment, the additional antibiotic is a beta-lactam antibiotic. In one aspect of this embodiment, the additional antibiotic is a penicillin-binding protein inhibitor.

### Definition of Terms

Unless stated to the contrary, the terms used in the Description and Claims have the following meanings.

The terms "hydrogen", "carbon" and "oxygen" in the compounds of the present invention compass all isotopes thereof. Isotopes should be understood to include those atoms having the same atomic number but different mass numbers. For example, isotopes of hydrogen include protium, tritium and deuterium, isotopes of carbon include ¹²C, ¹³C and ¹⁴C, and isotopes of oxygen include ¹⁶O and ¹⁸O.

The term "halogen" in the present invention means fluorine, chlorine, bromine or iodine. The term "halo" in the present invention refers to substitution by fluorine, chlorine, bromine or iodine.

The term "alkyl" in the present invention refers to a linear or branched saturated aliphatic hydrocarbon group, preferably a linear or branched group containing 1 to 6 carbon atoms, and more preferably a linear or branched group containing 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, etc. Alkyl may be substituted or unsubstituted, and when substituted, the substituent may be at any available point of attachment.

Both of the terms "carbonyl" and "acyl" in the present invention refer to -C(O)-.

The term "haloalkyl" in the present invention refers to an alkyl group substituted by at least one halogen.

The term "hydroxyalkyl" in the present invention refers to an alkyl group substituted by at least one hydroxy.

The term "alkoxy" in the present invention refers to -O-alkyl. Non-limiting examples of alkoxy include: methoxy, ethoxy, propoxy, n-propoxy, isopropoxy, isobutoxy, sec-butoxy, and the like. Alkoxy may be optionally substituted or unsubstituted, and when substituted, the substituent may be at any available point of attachment.

The term "cycloalkyl" in the present invention refers to a cyclic saturated hydrocarbon group. Suitable cycloalkyl may be substituted or unsubstituted monocyclic, bicyclic or tricyclic saturated hydrocarbon group with 3-12 carbon atoms, such as cyclopropyl, cyclobutyl, cyclopentyl and cyclohexyl.

The term "heterocyclyl" in the present invention refers to a 3- to 20-membered non-aromatic cyclic group ("3-20 membered heterocyclyl") having 1 to 4 ring heteroatoms (wherein each heteroatom is independently selected from the group consisting of nitrogen, oxygen, sulfur, boron, phosphorus and silicon). In heterocyclyl group containing one or more nitrogen atoms, the point of attachment may be a carbon or nitrogen atom, as long as the valence permits. Heterocyclyl group may be saturated or may be partially unsaturated. Each example of heterocyclyl may be optionally substituted or unsubstituted, and when substituted, the substituent may be at any available point of attachment.

The term "aryl" in the present invention refers to an aromatic system that can contain a single ring or a fused polycyclic ring, preferably an aromatic system containing a single ring or a fused bicyclic ring, which contains 6 to 18 carbon atoms, preferably about 6 to about 12 carbon atoms. Suitable aryl groups include, but are not limited to, phenyl, naphthyl, anthryl, tetrahydronaphthyl, fluorenyl and indanyl. Aryl may be optionally substituted or unsubstituted, and when substituted, the substituent may be at any available point of attachment.

The term "heteroaryl" in the present invention refers to an aryl group in which at least one carbon atom is replaced by a heteroatom, and is composed of 5-20 atoms (5-20 membered heteroaryl), more preferably composed of 5-12 atoms (5-12 membered heteroaryl), the heteroatoms being O, S, or N. The term "5-membered heteroaryl" in the present invention refers to a monocyclic aromatic system composed of 5 atoms, including, but not limited to, imidazolyl, pyrrolyl, furanyl, thienyl, pyrazolyl, oxazolyl, thiazolyl, isoxazolyl, isothiazolyl, oxadiazolyl and triazolyl. The terms "aryl-fused-heteroaryl" and "heteroaryl-fused-heteroaryl" in the present invention include, but are not limited to, indolyl, isoindolyl, benzopyrazolyl, benzimidazolyl, benzofuranyl, benzopyranyl, benzothienyl, benzoxazolyl, benzothiazolyl, benzisoxazolyl, benzisothiazolyl, quinolinyl, isoquinolinyl, quinazolinyl, dihydroquinazolinyl, cinnolinyl, quinoxalinyl, benzoxazinyl, benzothiazinyl, imidazopyridinyl, pyrimidopyrazolyl and pyrimido-imidazolyl. Heteroaryl may be optionally substituted or unsubstituted, and when substituted, the substituent may be at any available point of attachment.

The term "isomers" in the present invention are compounds that have the same molecular formula but differ in nature or in the bond sequence of their atoms or in the spatial arrangement of their atoms. Stereoisomers are isomers that differ in the spatial arrangement of their atoms. Stereoisomers that are not mirror images of each other are diastereomers; and stereoisomers that are non-overlapping mirror images of each other are enantiomers. When a compound has an asymmetric center, e.g., it is bonded to four different groups, a pair of enantiomers is possible. Enantiomers are characterized by the absolute configuration of their asymmetric centers and are described and designated as right-handed or left-handed (i.e. as (+) or (-)-isomer, respectively) through the R- and S-sequencing rules of Cahn and Prelog, or by the method of rotating the plane of polarized light by the molecule. Chiral compounds can exist as single enantiomers or mixtures thereof. A mixture containing equal proportion of enantiomers is called a "racemic mixture".

The term "pharmaceutically acceptable salt" in the present invention refers to a salt of the compound of the present invention, which is safe and effective when used in a mammalian body, and has desired biological activity.

The term "solvate" in the present invention conventionally refers to a complex formed by a combination of a solute (e.g., an active compound, a salt of an active compound) and a solvent (e.g., water). The solvent refers to a solvent which is known or easily determined by those skilled in the art. If it is water, the solvate is usually referred to as a hydrate, such as hemihydrate, monohydrate, dihydrate, trihydrate, or a substitution amount thereof.

The in vivo effects of a compound of formula (I) can be partly exerted by one or more metabolites formed in the body of the human or animal after the compound of formula (I) is administered. As mentioned above, the in vivo effects of a compound of formula (I) can also be exerted through the metabolism of a precursor compound ("prodrug"). The term "prodrug" in the present invention refers to a compound that is converted to a compound of formula (I) by reaction with an enzyme, gastric acid, etc., under physiological conditions in an organism, i.e. a compound that is converted to a compound of formula (I) by enzymatic oxidation, reduction, hydrolysis, etc., or converted to a compound of formula (I) by hydrolysis under gastric acid, etc.

The term "crystal" in the present invention refers to a solid whose internal structure is formed by regularly repeating constructive atoms (or groups thereof) in three dimensions, which is different from an amorphous solid that does not have such a regular internal structure.

The term "pharmaceutical composition" in the present invention refers to a mixture comprising any one of the compounds of the present invention, including corresponding isomer, prodrug, solvate, pharmaceutically acceptable salt or chemically protected form thereof, with one or more pharmaceutically acceptable carriers and/or one or more other drugs. The purpose of the pharmaceutical composition is to facilitate the administration of the compound to the organism. The composition is generally used to prepare a medicament for the treatment and/or prevention of a disease mediated by one or more kinases.

The term "pharmaceutically acceptable carrier" in the present invention refers to a carrier that does not cause a significant irritation to an organism and does not interfere with the biological activity and property of an administered compound, including all solvents, diluents or other excipients, dispersants, surfactants, isotonic agent, thickener or emulsifier, preservative, solid binder, lubricant, etc., unless any conventional carrier medium is incompatible with the compound of the present invention. Some examples of the pharmaceutically acceptable carrier include, but are not limited to, sugars, such as lactose, glucose and sucrose; starches, such as corn starch and potato starch; cellulose and its derivatives, such as sodium carboxymethyl cellulose, as well as cellulose and cellulose acetate; malt, gelatin, etc.

The term "excipient" in the present invention refers to an inert substance that is added to a pharmaceutical composition to further facilitate the administration of a compound. Excipients may include calcium carbonate, calcium phosphate, various sugars and various types of starch, cellulose derivatives, gelatin, vegetable oils and polyethylene glycol.

The term "infection" in the present invention can be caused by a variety of bacteria, which may be treated with a claimed drug alone or in combination with a penicillin binding protein inhibitor.

The term "subject suffered from bacterial infection" as used herein refers to an animal. In certain aspects, the animal is a mammal. The term individual also refers to, for example, primate (e.g., human), cattle, sheep, goat, horse, dog, cat, rabbit, rat, mouse, fish, bird, etc. In certain embodiments, the individual is human.

In one embodiment, the term "treatment" or "treating" as used herein refers to ameliorating a disease or disorder (i.e. slowing down or preventing or reducing the development of the disease or at least one clinical symptom thereof). In another embodiment, the term "treatment" or "treating" refers to reducing or improving at least one body parameter, including those that may not be recognized by the patient. In yet another embodiment, the term "treatment" or "treating" refers to regulating a disease or disorder bodily (e.g., stabilizing differentiable symptoms), physiologically (e.g., stabilizing body parameters), or both. In yet another embodiment, the term "treatment" or "treating" refers to preventing or delaying the onset or occurrence or progression of a disease or disorder.

### Specific Embodiments

The present invention will be further described in detail below in combination with examples, but the present invention is not limited thereto. The materials used in the following examples are commercially available unless otherwise specified.

### Example 1: (S)-3-((Z)-2-(((S)-2-(4-((1-(3-aminopropyl)-2-(azetidin-3-ylmethyl)-1H-pyrazol-2-ium-4-yl)carbamoyl)phenoxy)-1-carboxyethoxy)imino)-2-(2-aminothiazol-4-yl)acetylamino)-2,2-dimethyl-4-oxoazetidin-1-yl sulfate

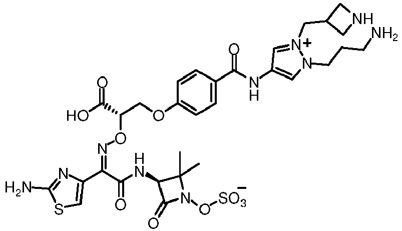

### Step 1: Preparation of tert-butyl (R)-(1,3-dihydroxy-3-methylbutan-2-yl)carbamate

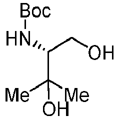

Under nitrogen protection, (tert-butoxycarbonyl)-D-serine methyl ester (70 g, 0.32 mol) was dissolved in tetrahydrofuran (2 L) at -78°C, followed by adding methyl magnesium bromide (500 mL, 0.166 mol, 3.0 M). After the addition, the mixture was warmed to room temperature and reacted at room temperature for 2 hours. After the reaction was complete as monitored by thin layer chromatography, saturated aqueous solution of ammonium chloride was added to quench the reaction. Ethyl acetate was added for extraction. The combined organic phase was washed with saturated brine, dried over anhydrous sodium sulfate, and filtered. The filtrate was concentrated to give the title product. ESI-MS m/z: 220.2 [M+H]⁺.

### Step 2: Preparation of (S)-2-((tert-butoxycarbonyl)amino)-3-hydroxy-3-methylbutanoic acid

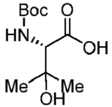

Tert-Butyl (*R*)-(1,3-dihydroxy-3-methylbutan-2-yl)carbamate (45 g, 0.20 mol) was dissolved in acetonitrile (800 mL) and a phosphate buffer (800 mL, 0.67 M, pH 6.8), followed by adding 2,2,6,6-tetramethylpiperidine oxide (3.1 g, 0.02 mol). The mixture was heated to 35°C, followed by adding sodium chlorite (46.6 g dissolved in 200 mL of water), and then adding a diluted solution of sodium hypochlorite (3 mL of purchased sodium hypochlorite solution dissolved in 100 mL of water). The reaction was carried out overnight at 35°C. After the reaction was complete as monitored by thin layer chromatography, the pH was adjusted to 3 with citric acid, followed by adding saturated aqueous solution of sodium chloride. Ethyl acetate was added for extraction. The combined organic phase was concentrated. An aqueous solution of sodium carbonate and ethyl acetate were added for extraction. The aqueous phase was cooled to 0°C and the pH was adjusted to 3 with 2.0 M nitric acid. Ethyl acetate was added for extraction. The combined organic phase was dried and concentrated to give the title product. ESI-MS m/z: 232.2 [M-H]⁻.

### Step 3: Preparation of tert-butyl (S)-(1-((benzyloxy)amino)-3-hydroxy-3-methyl-1-oxobutan-2-yl) carbamate

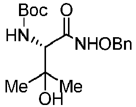

(5)-2-((tert-butoxycarbonyl)amino)-3-hydroxy-3-methylbutanoic acid (22 g, 94 mmol) was dissolved in N,N-dimethylformamide (250 mL), followed by adding 1-(3-dimethylaminopropyl)-3-ethylcarbodiimide hydrochloride (19.89 g, 103 mmol) and 1-hydroxybenzotriazole (14 g, 103 mmol). The mixture was stirred at room temperature for 30 minutes, followed by adding O-phenylhydroxylamine hydrochloride (16.5 g, 103 mmol) and sodium carbonate (30 g, 283 mmol). The reaction was carried out at room temperature for 24 hours. After the reaction was complete, water and ethyl acetate were added for extraction. The combined organic phase was dried and concentrated, and purified by silica gel column chromatography to give the title product ESI-MS m/z:339.2 [M+H]⁺.

### Step 4: Preparation of tert-butyl (5)-(N-benzyloxy-4,4-dimethylazetidin-2-on-3-yl)carbamate

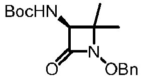

Tert-butyl (*S*)-(1-((benzyloxy)amino)-3-hydroxy-3-methyl-1-oxobutan-2-yl)carbamate (22 g, 65 mmol) was dissolved in pyridine (200 mL), followed by adding pyridine sulfur trioxide (13.4 g, 84 mmol) in batches at 0°C. After the addition, the mixture was heated to 55°C and reacted for 2 hours. The solvent was distilled off under reduced pressure, followed by adding a solution of potassium carbonate (54 g of potassium carbonate dissolved in 300 mL of water) and ethyl acetate (100 mL). The mixture was refluxed for 2 hours. After the reaction was complete, the mixture was cooled to room temperature, followed by adding ethyl acetate and water for extraction. The combined organic phase was dried and concentrated, and purified by silica gel column chromatography to give a white solid product. ESI-MS m/z: 321.2 [M+H]⁺.

### Step 5: Preparation of tert-butyl (S)-(1-hydroxy-2,2-dimethyl-4-oxoazetidin-3-yl)carbamate

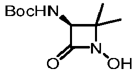

Tert-butyl (*S*)-(*N*-benzyloxy-4,4-dimethylazetidin-2-on-3-yl)carbamate (8.5 g, 26 mmol) was dissolved in methanol (90 mL), followed by adding 5% palladium on carbon (0.85 g, containing 50% water). The system was purged with hydrogen, and reaction was carried out at room temperature for 1 hour. After the reaction was complete, the palladium on carbon was filtered out. The solvent was removed by distillation under reduced pressure to give the title product. ESI-MS m/z: 231.1 [M+H]⁺.

### Step 6: Preparation of (S)-3-amino-2,2-dimethyl-4-oxoazetidin-1-yl bisulfate

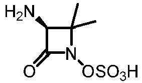

Tert-butyl (*S*)-(1-hydroxy-2,2-dimethyl-4-oxoazetidin-3-yl)carbamate (5 g, 21 mmol) was dissolved in pyridine (50 mL), followed by adding pyridine sulfur trioxide (3.9 g, 24 mmol) in batches at 0°C. After the addition, the mixture was heated to 55°C and reacted for 2 hours. The solvent was removed by distillation under reduced pressure. The solid was dissolved in saturated aqueous solution of potassium dihydrogen phosphate (400 mL). Dichloromethane was added for extraction. The aqueous phase was cooled to 0°C, followed by adding tetra-n-butylammonium bisulfate (8.5 g, 21 4 mmol). The reaction was carried out at 0-5°C for 1 hour. Dichloromethane was added for extraction. The organic phase was washed with saturated brine, dried over anhydrous sodium sulfate, and concentrated. To the obtained solid was added formic acid (50 mL). The reaction was carried out at room temperature for 4 hours, and a solid precipitated. Dichloromethane was added, and the mixture was cooled to 0-4°C. The solid was filtered, and vacuum dried to give a white solid product. ESI-MS m/z: 211.2 [M+H]⁺.

### Step 7: Preparation of ethyl 2-(2-((tert-butoxycarbonyl)amino)thiazol-4-yl)-2-oxoacetate

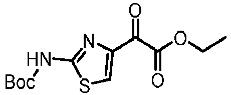

Ethyl 2-(2-aminothiazol-4-yl)-2-oxoacetate (10 g, 50 mmol) was dissolved in acetonitrile (250 mL), followed by adding di(tert-butyl) dicarbonate (23.2 mL, 100 mmol) and tetramethylethyldiamine (9.8 mL, 65 mmol). The reaction was carried out at room temperature for 3 hours. The solvent was removed by distillation under reduced pressure. 1 N hydrochloric acid and ethyl acetate were added for extraction. The organic phase was washed with saturated sodium bicarbonate and with saturated brine, dried over sodium sulfate, concentrated, and purified by column chromatography to give the white title product. ESI-MS m/z:301.2 [M+H]⁺.

### Step 8: Preparation of 2-(2-((tert-butoxycarbonyl)amino)thiazol-4-yl)-2-oxoacetic acid

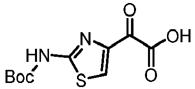

Ethyl 2-(2-((tert-butoxycarbonyl)amino)thiazol-4-yl)-2-oxoacetate (10 g, 50 mmol) was dissolved in tetrahydrofuran (150 mL)/methanol (50 mL), followed by adding an aqueous solution of sodium hydroxide (68 mL, 1 M, 68 mmol). The reaction was carried out at room temperature for 4 hours. After the reaction was complete, water and ethyl acetate were added for extraction. The pH of the combined aqueous phase was adjusted to 3 with 1 N hydrochloric acid. Water and ethyl acetate were added for extraction. The combined organic phase was dried and concentrated to give the white title product. ESI-MS m/z: 271.2

### Step 9: Preparation of tert-butoxycarbonyl-3-(4-nitro-1H-pyrazol-1-yl)-1-propylamine

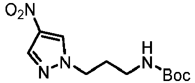

In a 500 mL single-neck flask, 4-nitropyrazole (10 g, 88.5 mmol) and tert-butoxycarbonyl-3-bromopropylamine (20.6 g, 86.6 mmol) were dissolved in N,N-dimethylformamide (150 mL), followed by adding potassium carbonate (30.5 g, 0.221 mol). The mixture was heated and stirred at 90°C overnight. After the reaction was complete, water and ethyl acetate were added for extraction. The combined organic phase was washed with saturated brine, dried over anhydrous sodium sulfate, and rotary evaporated to dryness to give a white crystal product. ESI-MS m/z: 271.2 [M+H]⁺.

### Step 10: Preparation of tert-butoxycarbonyl-3-(4-amino-1H-pyrazol-1-yl)-1-propylamine

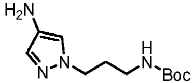

In a 100 mL single-neck flask, tert-butoxycarbonyl-3-(4-nitro-1*H*-pyrazol-1-yl)-1-propylamine (1.5 g, 5.6 mmol) was dissolved in methanol (20 mL), followed by adding wet 10% palladium on carbon (100 mg). A hydrogen balloon was connected to purge the system 3 times. The mixture was stirred at room temperature and reacted overnight. After the reaction was complete, the mixture was suction filtered, and the filtrate was rotary evaporated to dryness with silica gel, and subjected to silica gel column chromatography to give a red viscous product. ESI-MS m/z: 241.2 [M+H]⁺.

### Step 11: Preparation of tert-butoxycarbonyl-3-trifluoromethanesulfonyloxymethylazetidine

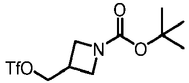

In a 100 mL three-neck flask, 3-hydroxyazetidine (1.5 g, 8 mmol) was dissolved in anhydrous dichloromethane (25 mL). The system was purged 3 times with argon, and cooled to -78°C. 2,6-Dimethylpyridine (1.4 mL, 12 mmol) and trifluoromethanesulfonic anhydride (1.62 mL, 9.6 mmol), and reacted for 1 hour while maintaining the temperature below -65°C. The reaction was monitored by TLC. After the reaction was complete, it was quenched by adding saturated aqueous solution of citric acid, and then adding saturated brine. The phases were separated, and the aqueous phase was extracted again with dichloromethane. The combined organic phase was dried over anhydrous sodium sulfate. The mixture was rotary evaporated to dryness and directly used in the next step.

### Step 12: Preparation of (S)-3-(4-bromophenoxy)-1,2-propanediol

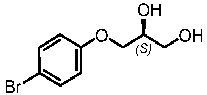

In a 1000 mL single-neck flask, p-bromophenol (133.6 g, 0.772 mol) and (*S*)-glycidol (60 g, 0.811 mol) were dissolved in absolute ethanol (250 mL), followed by adding triethylamine (5.4 mL, 39 mmol) as a catalyst. The reaction was carried out under reflux at 85°C for 7 hours. After the reaction was complete, the mixture was concentrated. 300 mL of dichloromethane was added for beating, followed by suction filteration. The filter cake was dried to give the title product as a white powder. ESI-MS m/z: 247.2 [M+H]⁺.

### Step 13: Preparation of (R)-3-(4-bromophenoxy)-2-hydroxypropionic acid

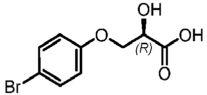

In a 2000 mL single-neck flask, (*S*)-3-(4-bromophenoxy)-1,2-propanediol (20 g, 81 mmol), 2,2,6,6-tetramethylpiperidine oxide (947 mg, 6.07 mmol), acetonitrile (300 mL), a phosphate buffer (pH 6.8, 0.67 M, 210 mL) and an aqueous solution of sodium chlorite (2.5 M, 97 mL) were add. A solution of sodium hypochlorite (0.5 mL commercially available solution containing 35% effective chlorine diluted with 15 mL of water) was added slowly and dropwise under stirring at 35°C. The reaction was carried out for 10 hours while maintaining at 35°C, monitored by TLC. After the reaction was complete, citric acid and ethyl acetate were added to quench the reaction, and the pH of the aqueous phase was 3. The phases were separated, and the aqueous phase was extracted twice with ethyl acetate. The combined organic phase was dried over anhydrous sodium sulfate, and rotary evaporated to dryness. The residue was dissolved in saturated solution of sodium carbonate. The mixture was extracted with an appropriate amount of ethyl acetate. The aqueous phase was adjusted to pH 3 with phosphoric acid, extracted 3 times with ethyl ester, dried, and rotary evaporated to dryness to give the title product as a light brown solid. ESI-MS m/z: 259.0

### Step 14: Preparation of tert-butyl (R)-3-(4-bromophenoxy)-2-hydroxypropionate

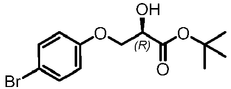

In a 1000 mL single-neck flask, (*R*)-3-(4-bromophenoxy)-2-hydroxypropionic acid (20 g, 77 mmol) and tert-butyl trichloroacetimidate (67 g, 307 mmol) were dissolved in tetrahydrofuran (300 mL), and were reacted for 2 days at 45°C under stirring. After the reaction was complete, the reaction liquid was rotary evaporated to dryness with silica gel, and subjected to silica gel column chromatography to give a white solid product. ESI-MS m/z: 315.0 ¹H NMR (400 MHz, CDCl₃) δ 7.43 - 7.35 (m, 2H), 6.86 - 6.77 (m, 2H), 4.39 (dt, *J* = 6.2, 3.1 Hz, 1H), 4.22 (qd, *J* = 9.7, 3.1 Hz, 2H), 3.24 (d, *J* = 6.2 Hz, 1H), 1.50 (s, 9H).

### Step 15: Preparation of tert-butyl (S)-3-(4-bromophenoxy)-2-((1,3-dioxoisoindolin-2-yl)oxy) propionate

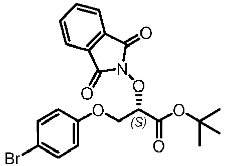

In a 100 mL single-neck flask, tert-butyl (*R*)-3-(4-bromophenoxy)-2-hydroxypropionat (1 g, 3.8 mmol), *N*-hydroxyphthalimide (1.25 g, 7.67 mmol) and triphenylphosphine (2 g, 7.63 mmol) were dissolved in anhydrous tetrahydrofuran (25 mL). The system was purged 3 times with argon. Diisopropyl azodicarboxylate (1.5 mL, 7.65 mmol) was added dropwise and the reaction was carried out overnight at room temperature under stirring. After the reaction was complete, the filtrate was concentrated and purified to give a pale yellow viscous product. ESI-MS m/z: 462.3 [M+H]⁺.

### Step 16: Preparation of (S)-4-(3-(tert-butoxy)-2-((1,3-dioxoisoindolin-2-yl)oxy)-3-oxopropoxy) benzoic acid

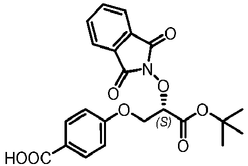

In a 100 mL single-neck flask, tert-butyl (*S*)-3-(4-bromophenoxy)-2-((1,3-dioxoisoindolin-2-yl)oxy) propionate (2.2 g, 4.76 mmol), palladium acetate (50 mg, 0.22 mmol), 4,5-bisdiphenylphosphino-9,9-dimethylxanthene (0.27 g, 0.47 mmol), formic acid (0.36 mL, 9.5 mmol), triethylamine (1.3 mL, 9.5 mmol), acetic anhydride (0.88 mL, 9.5 mmol) and tert-butanol (0.88 mL, 9.5 mmol) were dissolved in toluene (30 mL). The system was purged 3 times with argon. The mixture was heated to 90°C, and reacted overnight under stirring. After the reaction was complete, toluene was rotary evaporated. To the residue were added ethyl acetate and water, and the insoluble substance was filtered out. The filtrate was extracted with ethyl acetate. The combined organic phase was washed with saturated brine, dried over anhydrous sodium sulfate, concentrated and subjected to column chromatography to give a yellow solid product. ESI-MS m/z: 426.2

### Step 17: Preparation of tert-butyl (S)-3-(4-((1-(3-((tert-butoxycarbonyl)amino)propyl)-1H-pyrazol-4-yl)carbamoyl)phenoxy)-2-((1,3-dioxoisoindolin-2-yl)oxy)propionate

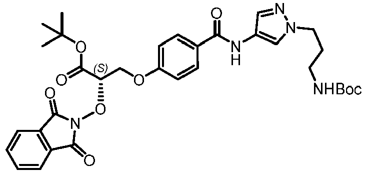

In a 100 mL single-neck flask, (*S*)-4-(3-(tert-butoxy)-2-((1,3-dioxoisoindolin-2-yl)oxy)-3-oxopropoxy)benzoic acid obtained in the previous step (1.8 g, 4.2 mmol), tert-butoxycarbonyl-3-(4-amino-1*H*-pyrazol-1-yl)-1-propylamine (1 g, 4.2 mmol), 2-(7-azobenzotriazolyl)-*N,N,N',N'*-tetramethyluronium hexafluorophosphate (1.93 g, 5.08 mmol) and N,N-diisopropylethylamine (1.35 mL, 8.4 mmol) were dissolved in anhydrous dichloromethane (30 mL), and reacted at room temperature for 5 h. After the reaction was complete, the organic phase was rotary evaporated to dryness. The mixture was extracted with ethyl acetate, and the combined organic phase was washed with saturated brine, dried over anhydrous sodium sulfate, concentrated, and subjected to silica gel column chromatography to give a yellow viscous product. ESI-MS m/z: 650.3 [M+H]⁺.

### Step 18: Preparation of (S)-4-(4-(3-(tert-butoxy)-2-((1,3-dioxoisoindolin-2-yl)oxy)-3-oxopropoxy) benzamido)-1-(3-((tert-butoxycarbonyl)amino)propyl)-2-((1-(tert-butoxycarbonyl)azetidin-3-y l)(methyl)-1H-pyrazol-2-ium trifluoromethanesulfonate

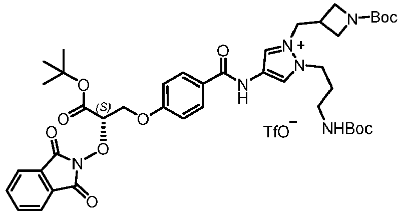

In a 100 mL single-neck flask, tert-butyl (S)-3-(4-((1-(3-((tert-butoxycarbonyl)amino)propyl) -1*H*-pyrazol-4-yl)carbamoyl)phenoxy)-2-((1,3-dioxoisoindolin-2-yl)oxy) propionate (1.05 g, 1.54 mmol, crude product), sodium bicarbonate (1.36 g, 15.4 mmol) and acetonitrile (50 mL) were mixed, and heated to 60°C. Newly prepared tert-butoxycarbonyl-3-trifluoromethanesulfonyloxy methylazetidine (2.7 g, 8.46 mmol) was added in batches within 1 h. The reaction was carried out for 3 h. After the reaction was complete, the mixture was suction filtered. The filtrate was rotary evaporated to dryness with silica gel, and subjected to column chromatography to give a light yellow viscous product. ESI-MS m/z: 819.9 [M+H]⁺.

### Step 19: Preparation of (S)-4-(4-(2-(aminooxy)-3-(tert-butoxy)-3-oxopropoxy)benzamido)-1-(3-((tert-butoxycarbonyl)amino)propyl)-2-((1-(tert-butoxycarbonyl)azetidin-3-yl)methyl)-1H-pyra zol-2-ium trifluoromethanesulfonate

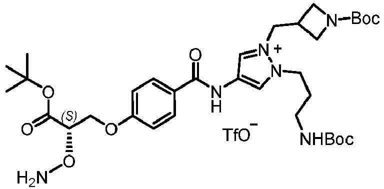

In a 50 mL single-neck flask, (*S*)-4-(4-(3-tert-butoxy-2-((1,3-dioxoisoindolin-2-yl)oxy)-3-oxopropoxy)benzamido)-1-(3-((tert-butoxycarbonyl)amino)propyl)-2-((1-(tert-butoxycarbonyl) azetidin-3-yl)methyl)-1*H*-pyrazol-2-ium trifluoromethanesulfonate (1.04 g, 1.07 mmol) was dissolved in methanol (10 mL), followed by adding 7 N ammonia-methanol solution (1.2 mL, 9.6 mmol). The reaction was carried out at room temperature for 2 days. After the reaction was complete, the mixture was rotary evaporated to dryness with silica gel, and subjected to column chromatography to give a light yellow viscous solid product. ESI-MS m/z: 689.8[M+H]⁺.

### Step 20: Preparation of (S,Z)-4-(4-(3-(tert-butoxy)-2-((((2-((tert-butoxycarbonyl)amino) thiazol-4-yl)(carboxy)methylene)amino)oxy)-3-oxopropoxy)benzamido)-1-(3-((tert-butoxycarb onyl)amino)propyl)-2-((1-(tert-butoxycarbonyl)azetidin-3-yl)methyl)-1H-pyrazol-2-ium trifluoromethanesulfonate

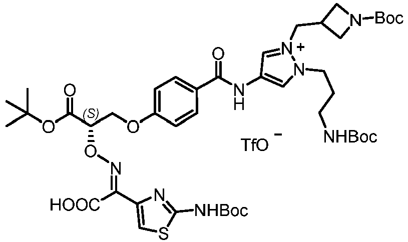

In a 50 mL single-neck flask, (*S*)-4-(4-(2-(aminooxy)-3-(tert-butoxy)-3-oxopropoxy)benzamido)-1-(3-((tert-butoxycarbonyl)amino)propyl)-2-((1-(tert-butoxycarbonyl)azetidin-3-yl)methyl)-1*H-*pyrazol-2-ium trifluoromethanesulfonate (375 mg, 0.45 mmol) and previously prepared 2-(2-((tert-butoxycarbonyl)amino)thiazol-4-yl)-2-oxoacetic acid (128 mg, 0.47 mmol) were dissolved in dichloromethane (6 mL) and methanol (14 mL), and reacted at room temperature for 2 h under stirring. After the reaction was complete, the mixture was directly rotary evaporated to dryness to give a light yellow solid product. ESI-MS m/z: 943.5 [M+H]⁺.

### Step 21: Preparation of (S)-3-((Z)-2-((((S)-1-(tert-butoxy)-3-(4-((1-(3-((tert-butoxycarbonyl)amino) propyl)-2-((1-(tert-butoxycarbonyl)azetidin-3-yl)methyl)-1H-pyrazol-2-ium-4-yl)carbamoyl) phenoxy)-1-oxopropan-2-yl)oxy)imino)-2-(2-((tert-butoxycarbonyl)amino)thiazol-4-yl) acetylamido)-2,2-dimethyl-4-oxoazetidine-1-yl sulfate

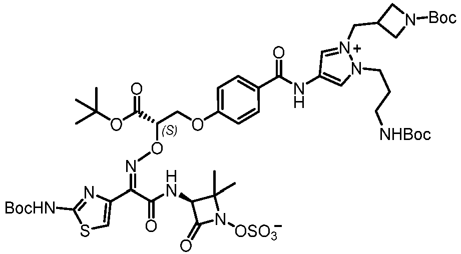

In a 100 mL single-neck flask, (*S*,*Z*)-4-(4-(3-(tert-butoxy)-2-((((2-((tert-butoxycarbonyl)amino) thiazol-4-yl)(caboxy)methylene)amino)oxy)-3-oxopropoxy)benzamido)-1-(3-((tert-butoxycarb onyl)amino)propyl)-2-((1-(tert-butoxycarbonyl)azetidin-3-yl)methyl)-1*H*-pyrazol-2-ium trifluoromethanesulfonate (456 mg, 0.42 mmol), tripyrrolidinylphosphonium bromide hexafluorophosphate (252 mg, 0.54 mmol) and (*S*)-3-amino-2,2-dimethyl-4-oxoazetidin-1-yl bisulfate (92 mg, 0.44 mmol) were dissolved in acetonitrile (15 mL), followed by adding dropwise N,N-diisopropylethylamine in ethyl acetate (140 µL, 0.8 mmol). The reaction was carried out overnight at room temperature under stirring. After the reaction was complete, the mixture was concentrated and subjected to column chromatography to give the title product. ESI-MS m/z: 1135.6 [M+H]⁺.

### Step 22: Preparation of (S)-3-((Z)-2-(((S)-2-(4-((1-(3-aminopropyl)-2-(azetidin-3-ylmethyl)-1H-pyrazol-2-ium-4-yl)carbamoyl)phenoxy)-1-carboxyethoxy)imino)-2-(2-aminothiazol-4-yl)acety lamino)-2,2-dimethyl-4-oxoazetidin-1-yl sulfate

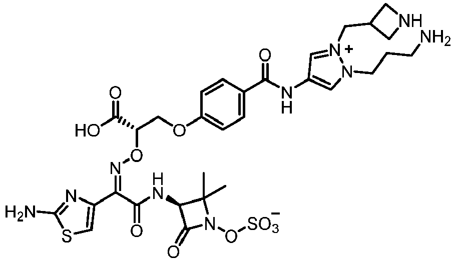

In a 25 mL single-neck flask, (*S*)-3-((*Z*)-2-((((*S*)-1-(tert-butoxy)-3-(4-((1-(3-((tert-butoxycarbonyl) amino)propyl)-2-((1-(tert-butoxycarbonyl)azetidin-3-yl)methyl)-1*H*-pyrazol-2-ium-4-yl) carbamoyl)phenoxy)-1-oxopropan-2-yl)oxy)imino)-2-(2-((tert-butoxycarbonyl)amino)thiazol-4 -yl)acetylamido)-2,2-dimethyl-4-oxoazetidin-1-yl sulfate (90 mg, 79 µmol) was dissolved in dichloromethane (3 mL). Trifluoroacetic acid (3 mL) was added dropwise in an ice bath, and the reaction was carried out for 2 h in an ice bath. After the reaction was complete, the mixture was concentrated at room temperature, prepared, and lyophilized to give the title compound. ¹H NMR (400 MHz, D₂O) δ 8.54 - 8.49 (m, 1H), 8.43 (t, *J* = 3.2 Hz, 1H), 7.78 - 7.69 (m, 2H), 6.97 (dd, *J* = 15.6, 8.5 Hz, 3H), 5.04 (dd, *J* = 5.3, 2.2 Hz, 1H), 4.73 (d, *J* = 7.6 Hz, 2H), 4.55 - 4.41 (m, 5H), 4.21 - 4.12 (m, 2H), 4.01 (dd, *J* = 11.7, 7.5 Hz, 2H), 3.52 (s, 1H), 3.05 - 2.97 (m, 2H), 2.27 - 2.17 (m, 2H), 1.30 (s, 3H), 0.91 (s, 3H). ESI-MS m/z: 779.9 [M+H]⁺.

### Example 2: (S)-3-((Z)-2-(((S)-2-(4-(1-(3-aminopropyl)-2-(azetidin-3-ylmethyl)-1H-pyrazol-2-ium-4-carboxamido)phenoxy)-1-carboxyethoxy)imino)-2-(2-aminothiazol-4-yl)acetylamido)-2,2-dimethyl-4-oxoazetidin-1-yl sulfate

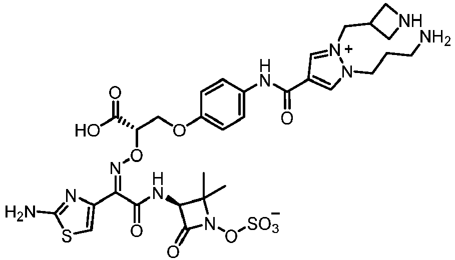

### Step 1: Preparation of tert-butyl (R)-3-(4-nitrophenoxy)-2-hydroxypropionate

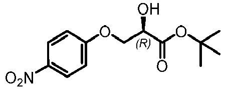

The title compound was prepared by a preparation method which was the same as the preparation method of steps 12-14 in Example 1, except that p-bromophenol was replaced with p-nitrophenol.

### Step 2: Preparation of tert-butyl (R)-3-(4-aminophenoxy)-2-hydroxypropionate

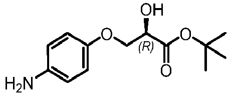

In a 100 mL single-neck flask, tert-butyl (*R*)-3-(4-bromophenoxy)-2-hydroxypropionate (3.1 g, 11 mmol) was dissolved in methanol (85 mL), followed by adding wet 10% palladium on carbon (320 mg). The system was purged 3 times with hydrogen. The reaction was carried out for 6 h at room temperature under stirring. After the reaction was complete, the mixture was suction filtered. The filtrate was rotary evaporated to dryness with silica gel, and subjected to silica gel column chromatography to give the off-white title product. ESI-MS m/z: 254.2 [M+H]⁺.

### Step 3: Preparation of tert-butyl (R)-3-(4-(1-(3-((tert-butoxycarbonyl)amino)propyl)-1H-pyrazole-4-carboxamido)phenoxy)-2-hydroxypropionate

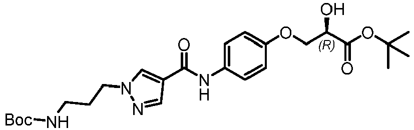

In a 100 mL single-neck flask, tert-butyl (*R*)-3-(4-aminophenoxy)-2-hydroxypropionate (207 mg, 0.82 mmol) obtained in the previous step, 1-(3-tert-butoxycarbonyl-aminopropyl)-1*H*-pyrazole-4-carboxylic acid (220 mg, 0.82 mmol), 2-(7-azobenzotriazolyl)-*N,N,N',N'*-tetramethyluronium hexafluorophosphate (373 mg, 0.98 mmol) and *N,N*-diisopropylethylamine (0.27 mL, 1.6 mmol) were dissolved in anhydrous *N,N-*dimethylformamide (6 mL) and reacted at room temperature for 2.5 h. After the reaction was complete, ethyl acetate and water were added for extraction. The combined organic phase was washed with saturated brine, dried over anhydrous sodium sulfate, and concentrated to give the yellow viscous title product. ESI-MS m/z: 505.3 [M+H]⁺.

### Step 4: Preparation of tert-butyl (S)-3-(4-(1-(3-((tert-butoxycarbonyl)amino)propyl)-1H-pyrazole-4-carboxamido)phenoxy)-2-((1,3-dioxoisoindolin-2-yl)oxy)propionate

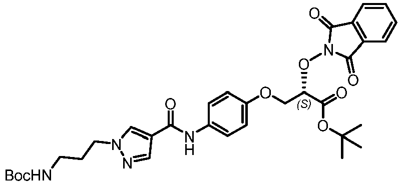

In a 100 mL single-neck flask, tert-butyl (*R*)-3-(4-(1-(3-((tert-butoxycarbonyl)amino)propyl)-1*H-*pyrazole-4-carboxamido)phenoxy)-2-hydroxypropionic acid prepared in the previous step, N-hydroxyphthalimide (1.25 g, 7.67 mmol) and triphenylphosphine (2 g, 7.63 mmol) were dissolved in anhydrous tetrahydrofuran (25 mL). The system was purged 3 times with argon. Diisopropyl azodicarboxylate (1.5 mL, 7.65 mmol) was added dropwise. The reaction was carried out at room temperature overnight under stirring. After the reaction was complete, the filtrate was concentrated to give the pale yellow viscous title product. ESI-MS m/z: 650.3 [M+H]⁺.

### Step 5: (S)-3-((Z)-2-(((S)-2-(4-(1-(3-aminopropyl)-2-(azetidin-3-ylmethyl)-1H-pyrazol-2-ium-4-carboxamido)phenoxy)-1-carboxyethoxy)imino)-2-(2-aminothiazol-4-yl)acetylamido)-2,2-dimethyl-4-oxoazetidine-1-yl sulfate

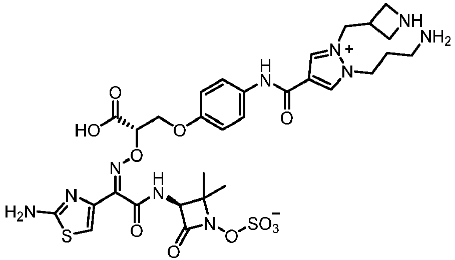

The title compound was prepared by a preparation method which was the same as steps 18-22 of Example 1, except that the raw material tert-butyl (*S*)-3-(4-((1-(3-((tert-butoxycarbonyl) amino)propyl)-1*H*-pyrazol-4-yl)carbamoyl) phenoxy)-2-((1,3-dioxoisoindolin-2-yl)oxy)propionate was replaced with tert-butyl (*S*)-3-(4-(1-(3-((tert-butoxycarbonyl)amino)propyl)-1*H*-pyrazole-4-carboxamido)phenoxy)-2-((1,3-dioxoisoindolin-2-yl)oxy)propionate. ¹H NMR (400 MHz, D₂O) δ 8.40 - 8.50 (m, 2H), 8.43 (t, *J* = 3.2 Hz, 1H), 7.18 (m, 2H), 6.88 (m, 3H), 5.04 (dd, *J* = 5.3, 2.2 Hz, 1H), 4.68 (d, *J* = 7.6 Hz, 2H), 4.59 - 4.46 (m, 5H), 4.23 - 4.16 (m, 2H), 4.08 (dd, *J* = 11.8, 7.5 Hz, 2H), 3.56 (s, 1H), 3.04 - 2.80 (m, 2H), 2.33 - 1.55 (m, 2H), 1.61-1.20 (s, 2H), 0.91 (s, 3H). ESI-MS m/z: 779.9 [M+H]⁺.

### Example 3: (3S)-3-((Z)-2-(((1S)-2-(4-(2-(3-aminopropoxy)-2,3-dihydro-1H-pyrazolo[1,2-a]pyrazol-4-ium-6-yl)phenoxy)-1-carboxyethoxy)imino)-2-(2-aminothiazol-4-yl)acetylamino)-2,2-dimeth yl-4-oxoazetidin-1-yl sulfate

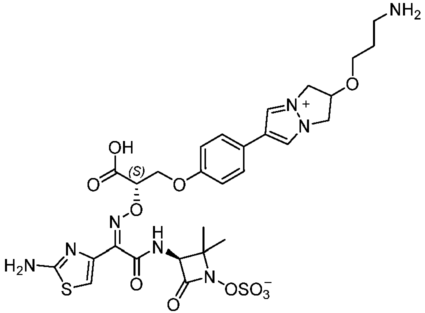

### Step 1: Preparation of 6-bromo-2-hydroxy-2,3-dihydro-1H-pyrazolo[1,2-a]pyrazol-4-ium bromide

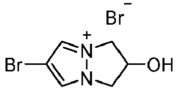

In a 100 mL single-neck flask, 4-bromopyrazole (2.94 g, 20 mmol) and epibromohydrin (2.74 g, 20 mmol) were dissolved in DMF (30 mL), and heated and stirred at 100°C overnight. After the reaction was complete, most of N,N-dimethylformamide was rotary evaporated. Methyl tert-butyl ether was added, and a yellow-white solid precipitated out. The title product was obtained by suction filtration and drying. ESI-MS m/z: 204.1 [M]⁺.

### Step 2: Preparation of 6-bromo-2-(3-((tert-butoxycarbonyl)amino)propoxy)-2,3-dihydro-1H-pyrazolo[1,2-a]pyrazol-4-ium

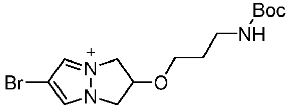

In a 100 mL three-neck flask, 6-bromo-2-hydroxy-2,3-dihydro-1*H*-pyrazolo[1,2-a]pyrazol-4-ium bromide (100 mg, 0.4 mmol) was dissolved in anhydrous *N,N-*dimethylformamide (5 mL), and cooled to 0-5°C in an ice bath. Sodium hydride (17 mg, 0.41 mmol) was added and the mixture was stirred for 30 minutes. N-tert-butoxycarbonyl-3-aminopropyl bromide (88 mg, 0.37 mmol) was added. The reaction was continued for 3 hours in an ice bath, monitored by TLC. After the reaction was complete, the solvent was rotary evaporated with silica gel, and the residue was subjected to silica gel column chromatography to give the title product. ESI-MS m/z: 361.08

### Step 3: Preparation of 6-(4-((R)-3-(tert-butoxy)-2-hydroxy-3-oxopropoxy)phenyl)-2-(3-((tert-butoxycarbonyl)amino)propoxy)-2,3-dihydro-1H-pyrazolo[1,2-a]pyrazol-4-ium

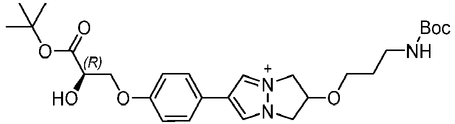

In a 250 mL single-neck flask, 6-bromo-2-(3-((tert-butoxycarbonyl)amino)propoxy)-2,3-dihydro-1H-pyrazolo[1,2-a]pyrazol-4-ium (3.4 g, 9.48 mmol), tert-butyl (R)-2-hydroxy-3-(4-(4,4,5,5-tetramethyl-1,3,2-dioxaborolan-2-yl)phenoxy)propionate (4.09 g, 7.9 mmol), [1,1'-bis(diphenylphosphino)ferrocene]palladium dichloride (0.58 g, 0.79 mmol) and potassium phosphate (5 g, 23.7 mmol) were dissolved in a mixed solvent of dioxane (60 ml) and water (15 ml). Under argon protection, the mixture was heated and stirred overnight at 100°C. The reaction was monitored by TLC. After the reaction was complete, the mixture was extracted with ethyl acetate, rotary evaporated to dryness with silica gel, and subjected to silica gel column chromatography to give a gray-black solid product. ESI-MS m/z: 518.6 [M+H]⁺.

### Step 4: Preparation of 6-(4-((S)-3-(tert-butoxy)-2-((1,3-dioxoisoindolin-2-yl)oxy)-3-oxopropoxy) phenyl)-2-(3-((tert-butoxycarbonyl)amino)propoxy)-2,3-dihydro-1H-pyrazolo[1,2-a]pyrazol-4-i um

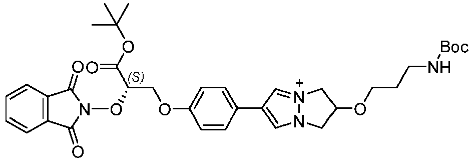

In a 100 mL three-neck flask, 6-(4-((*R*)-3-(tert-butoxy)-2-hydroxy-3-oxopropoxy)phenyl)-2-(3-((tert-butoxycarbonyl)amino)propoxy)-2,3-dihydro-1*H*-pyrazolo[1,2-a]pyrazol-4-ium (1.87 g, 3.61 mmol), *N*-hydroxyphthalimide (1.17 g, 7.21 mmol) and triphenylphosphine (1.89 g, 7.21 mmol) were dissolved in anhydrous tetrahydrofuran (30 ml). Under argon protection, diisopropyl azodicarboxylate (1.45 g, 7.21 mmol) was added. The reaction was carried overnight at room temperature, monitored by TLC. After the reaction was complete, the mixture was extracted with ethyl acetate, rotary evaporated to dryness with silica gel, and subjected to silica gel column chromatography to give an off-white solid product. ESI-MS m/z: 663.8 [M+H]⁺.

### Step 5: Preparation of (3S)-3-((Z)-2-(((1S)-2-(4-(2-(3-aminopropoxy)-2,3-dihydro-1H-pyrazolo [1,2-a]pyrazol-4-ium-6-yl)phenoxy)-1-carboxyethoxy)imino)-2-(2-aminothiazol-4-yl)acetylami no)-2,2-dimethyl-4-oxoazetidin-1-yl sulfate

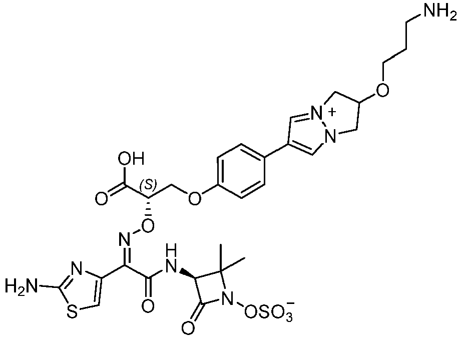

The title compound was prepared by a preparation method which was the same as the preparation method of steps 19-22 in Example 1, except that the raw material (*S*)-4-(4-(3-tert-butoxy-2-((1,3-dioxoisoindolin-2-yl)oxy)-3-oxopropoxy)benzamido)-1-(3-((tert-butoxycarbonyl)amino)propyl)-2-((1-(tert-butoxycarbonyl)azetidin-3-yl)methyl)-1*H*-pyrazol-2-ium trifluoromethanesulfonate was replaced with 6-(4-((*S*)-3-(tert-butoxy)-2-((1,3-dioxoisoindolin-2-yl)oxy)-3-oxopropoxy)phenyl)-2-(3-((tert-butoxycarbonyl)amino)propoxy)-2, 3-dihydro-1H-pyrazolo[1,2-a]pyrazol-4-ium. ¹H NMR (400 MHz, D₂O) δ 8.73 (s, 2H), 7.63 (d, *J* = 8.2 Hz, 2H), 7.01-7.03 (d, *J* = 8.3 Hz, 2H), 6.83 (s, 1H), 4.99 (d, *J* = 4.5 Hz, 2H), 4.74-4.60 (m, 6H), 4.42-4.35 (m, 2H), 3.61 (t, *J* = 6.2 Hz, 1H), 2.83 (t, *J* = 8.2, 2H), 1.82 - 1.77 (m, 2H), 1.32 (s, 3H), 1.04 (s, 3H). ESI-MS m/z: 722.6[M+H]⁺.

### Example 4: (S)-3-((Z)-2-(2-aminothiazol-4-yl)-2-(((S)-1-carboxy-2-(4-(6-((S)-2,6-diaminohexanamido)-1-methylpyridin-1-ium-3-yl)phenoxy)ethoxy)imino)acetylamino)-2,2-dimethyl-4-oxoazetidin-1-yl sulfate

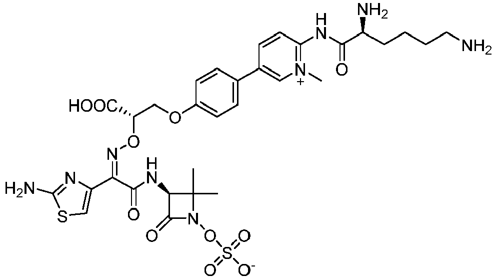

### Step 1: Preparation of di(tert-butyl) (6-((5-bromopyridin-2-yl)amino)-6-oxohexan-1,5-diyl) (S)-dicarbamate

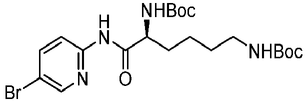

(S)-2,6-Di(tert-butoxycarbonylamino)caproic acid (4 g, 11.56 mmol), dichloromethane (60 ml), *N,N*-diisopropylethylamine (3 g, 23.12 mmol), 2-(7-oxobenzotriazolyl)-*N,N,N',N'-*tetramethyluronium hexafluorophosphate (5.27 g, 13.87 mmol) and 2-amino-5-bromopyridine (2 g, 11.56 mmol) were sequentially added and reacted overnight under reflux. After the reaction was complete, the mixture was concentrated to dryness. The residue was dissolved in ethyl acetate, washed sequentially with 10% solution of citric acid, saturated solution of sodium bicarbonate, and saturated brine, dried over anhydrous sodium sulfate, concentrated, and subjected to silica gel column chromatography to give 4.20 g of colorless oil. ESI-MS m/z:501.1 [M+H]⁺.

### Step 2 Preparation of di(tert-butyl) (6-oxo-6-((5-(4,4,5,5-tetramethyl-1,3,2-dioxaborolan-2-yl) (pyridin-2-yl)amino)hexan-1,5-diyl)(S)-dicarbamate

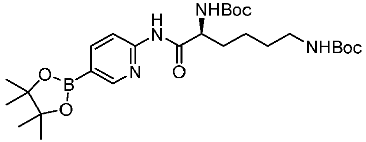

Di(tert-butyl) (6-((5-bromopyridin-2-yl)amino)-6-oxohexan-1,5-diyl)(*S*)-dicarbamate (2 g, 3.99 mmol), bis(pinacolato)diboron (1.52 g, 5.98 mmol), [1,1'-bis(diphenylphosphino)ferrocene] palladium dichloride (290 mg, 0.40 mmol), potassium acetate (1.17 g, 11.97 mmol) and 1,4-dioxane (30 ml) were sequentially added to a 100 ml single-neck flask. The system was purged 3 times with nitrogen. Under nitrogen protection, the reaction was carried overnight at 90°C. After the reaction was complete, 30 ml of ethyl acetate was added for beating for 5 minutes. The mixture was filtered through celite, concentrated, and subjected to column chromatography to give 1.80 g of the title compound. ESI-MS m/z:549.3 [M+H]⁺.

### Step 3: Preparation of tert-butyl (R)-3-(4-(6-((5)-2,6-bis((tert-butoxycarbonyl)amino)hexanamido) pyridin-3-yl)phenoxy)-2-hydroxypropionate

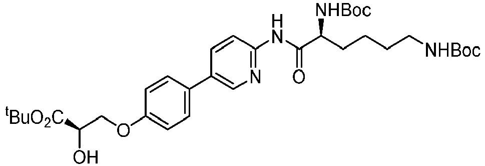

Tert-butyl (*R*)-3-(4-bromophenoxy)-2-hydroxypropionate (1 g, 3.15 mmol), di(tert-butyl) (6-oxo-6-((5-(4,4,5,5-tetramethyl-1,3,2-dioxaborolan-2-yl)pyridin-2-yl)amino)hexan-1,5-diyl)(*S*) -dicarbamate (2.25 g, 4.10 mmol), [1,1'-bis(diphenylphosphino)ferrocene]palladium dichloride (230 mg, 0.32 mmol), cesium carbonate (2.05 g, 6.31 mmol), 1,4-dioxane (20 ml) and water (4 ml) were sequentially added to a 100 ml single-neck flask. The system was purged 3 times with nitrogen. The reaction was carried out overnight at 90°C under nitrogen protection. After the reaction was complete, ethyl acetate and water were added. The mixture was filtered with celite, and extracted 3 times with ethyl acetate. The combined organic phase was washed twice with saturated brine, dried over anhydrous sodium sulfate, concentrated, and subjected to column chromatography to give 1.70 g of brown oil,. ESI-MS m/z:659.3 [M+H]⁺.

### Step 4 Preparation of tert-butyl (S)-3-(4-(6-((S)-2,6-bis((tert-butoxycarbonyl)amino)hexanamido) pyridin-3-yl)phenoxy)-2-((1,3-dioxoisoindolin-2-yl)oxy)propionate

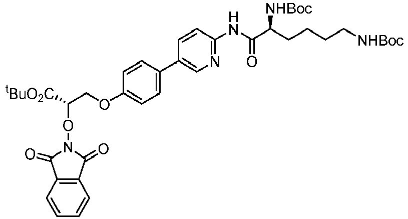

Tert-butyl (*R*)-3-(4-(6-((*S*)-2,6-bis((tert-butoxycarbonyl)amino)hexanamido)pyridin-3-yl) phenoxy)-2-hydroxypropionate (1.7 g, 2.58 mmol), *N*-hydroxyphthalimide (547 mg, 3.35 mmol), triphenylphosphine (1.02 mg, 3.87 mmol) and anhydrous tetrahydrofuran (30 ml) were added to a 100 ml three-neck flask. The system was cooled to 0°C, and diisopropyl azodicarboxylate (0.783 mg, 3.87 mmol) was added dropwise. After the addition, the system was allowed to naturally warm to room temperature and the reaction was carried out overnight. The disappearance of the raw materials was monitored. After the reaction was complete, the mixture was directly concentrated with silica gel, and subjected to column chromatography to give 1.67 g of the title compound. ESI-MS m/z:804.3 [M+H]⁺.

### Step 5: Preparation of 2-((S)-2,6-bis((tert-butoxycarbonyl)amino)hexanamido)-5-(4-((S)-3-(tert-butoxy)-2-((1,3-dioxoisoindolin-2-yl)oxy)-3-oxopropoxy)phenyl)-1-methylpyridin-1-ium iodide

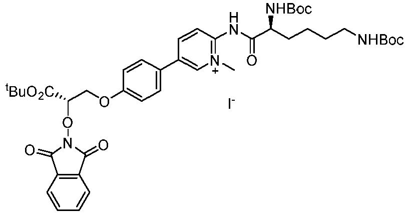

Tert-butyl (*S*)-3-(4-(6-((*S*)-2,6-bis((tert-butoxycarbonyl)amino)hexanamido)pyridin-3-yl)phenoxy)-2-((1,3-dioxoisoindolin-2-yl)oxy)propionate (1.17 g, 1.46 mmol), acetonitrile (12 ml) and methyl iodide (2.07 g, 14.55 mmol) were added to a 50 ml single-neck flask. The reaction was carried out under reflux for 1 day. After the reaction was complete, the mixture was concentrated with silica gel, and subjected to column chromatography to give 860 mg of the title compound. ESI-MS m/z:818.3 [M-I⁻]⁺.

### Step 6: Preparation of 5-(4-((S)-2-(aminooxy)-3-(tert-butoxy)-3-oxopropoxy)phenyl)-2-((S)-2,6-bis((tert-butoxycarbonyl)amino)hexanamido)-1-methylpyridin-1-ium iodide

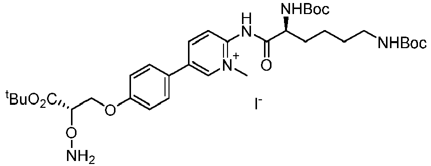

2-((*S*)-2,6-bis((tert-butoxycarbonyl)amino)hexanamido)-5-(4-((*S*)-3-(tert-butoxy)-2-((1,3-dioxo isoindolin-2-yl)oxy)-3-oxopropoxy)phenyl)-1-methylpyridin-1-ium iodide (860 mg) was dissolved in ethanol (10 ml), followed by adding hydrazine hydrate (1 ml). The reaction was carried out at room temperature for 2 h. After the reaction was complete, the mixture was concentrated to remove ethanol and hydrazine hydrate to give brown oil, to which was added dichloromethane for beating for 10 minutes, during which a white solid precipitated out. The mixture was filtered, and the filtrate was concentrated and subjected to column chromatography (dichloromethane : ethyl acetate = 3:1, containing 1% methanol) to give 620 mg of light yellow oil. ESI-MS m/z:688.3 [M-I⁻]⁺.

### Step 7: Preparation of 2-((S)-2,6-bis((tert-butoxycarbonyl)amino)hexanamido)-5-(4-((S)-3-(tert-butoxy)-2-((((Z)-(2-((tert-butoxycarbonyl)amino)thiazol-4-yl)(carboxy)methylene)amino) oxy)-3-oxopropoxy)phenyl)-1-methylpyridin-1-ium iodide

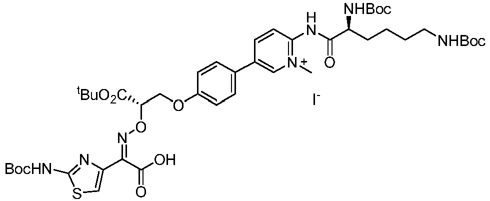

5-(4-((*S*)-2-(aminooxy)-3-(tert-butoxy)-3-oxopropoxy)phenyl)-2-((*S*)-2,6-bis((tert-butoxycarbo nyl)amino)hexanamido)-1-methylpyridin-1-ium iodide (240 mg, 0.35 mmol) was dissolved in a mixture of ethanol (4 ml) and 1,2-dichloroethane, followed by adding 2-(2-((tert-butoxycarbonyl)amino)thiazol-4-yl)-2-oxoacetic acid (114 mg, 0.42 mmol). The reaction was carried out at room temperature for 2 h. After the raw materials disappeared and the reaction was complete, the mixture was concentrated with silica gel, and subjected to column chromatography to give 166 mg of the title compound. ESI-MS m/z:942.4 [M-I⁻]⁺.

### Step 8: Preparation of 2-((S)-2,6-bis((tert-butoxycarbonyl)amino)hexanamido)-5-(4-((S)-3-(tert-butoxy)-2-((((Z)-1-(2-((tert-butoxycarbonyl)amino)thiazol-4-yl)-2-(((S)-2,2-dimethyl-4-ox o-1-(sulfonyloxy)azetidin-3-yl)amino)-2-oxoethylene)amino)oxy)-3-oxopropoxy)phenyl)-1-methyl pyridin-1-ium iodide

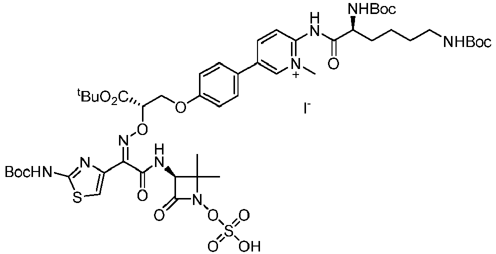

2-((*S*)-2,6-bis((tert-butoxycarbonyl)amino)hexanamido)-5-(4-((*S*)-3-(tert-butoxy)-2-((((*Z*)-(2-((t ert-butoxycarbonyl)amino)thiazol-4-yl)(carboxy)methylene)amino)oxy)-3-oxopropoxy)phenyl) -1-methylpyridin-1-ium iodide (166 mg, 0.18 mmol) was dissolved in *N,N-*dimethylformamide (3 ml), followed by adding (*S*)-3-amino-2,2-dimethyl-4-oxoazetidin-1-yl bisulfate (48 mg, 0.23 mmol), *N,N*-diisopropylethylamine (46 mg, 0.35 mmol) and 2-(7-oxobenzotriazolyl)-*N,N,N',N*'-tetramethyluronium hexafluorophosphate (100 mg, 0.26 mmol). The reaction was carried at room temperature for 2 h. After the raw materials disappeared and the reaction was complete, the reaction liquid was directly subjected to reversed-phase column chromatography and lyophilization to give 150 mg of the title compound. ESI-MS m/z:1134.4 [M-1⁻]⁺.

### Step 9: Preparation of (S)-3-((Z)-2-(2-aminothiazol-4-yl)-2-(((S)-1-carboxy-2-(4-(6-((S)-2,6-diaminohexanamido)-1-methylpyridin-1-ium-3-yl)phenoxy)ethoxy)imino)acetylamino)-2,2-dimethyl-4-oxoazetidin-1-yl sulfate

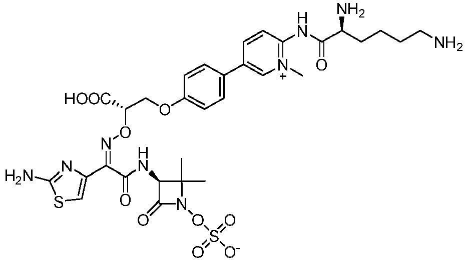

2-((*S*)-2,6-bis((tert-butoxycarbonyl)amino)hexanamido)-5-(4-((*S*)-3-(tert-butoxy)-2-((((*Z*)-1-(2-((tert-butoxycarbonyl)amino)thiazol-4-yl)-2-(((*S*)-2,2-dimethyl-4-oxo-1-(sulfonyloxy)azetidin-3 -yl)amino)-2-oxoethylene)amino)oxy)-3-oxopropoxy)phenyl)-1-methylpyridin-1-ium iodide (150 mg, 0.13 mmol) was dissolved in dichloromethane (5 ml), followed by sequentially adding a catalytic amount of triethylsilane and trifluoroacetic acid (2 ml). The reaction was carried out at room temperature for 2 h. After the reaction was complete, the mixture was concentrated. The residue was dissolved in a mixed solvent of acetonitrile and water (volume ratio 1:1), and subjected to reversed-phase column chromatography and lyophilization to give 25 mg of the title compound. ESI-MS m/z:778.2 [M+H]⁺. ¹H NMR (400 MHz, DMSO*-*d₆+D₂O) δ 8.44 (s, 1H), 8.28 - 8.19 (m, 1H), 8.13 - 8.03 (m, 1H), 7.61 - 7.43 (m, 2H), 7.11 - 6.93 (m, 2H), 6.84 (s, 1H), 4.81 - 4.73 (m, 1H), 4.71 (s, 1H), 4.44 - 4.21 (m, 2H), 3.81 (s, 3H), 3.71 - 3.65 (m, 1H), 2.92 - 2.70 (m, 2H), 1.96 - 1.72 (m, 2H), 1.65 - 1.54 (m, 2H), 1.53 - 1.44 (m, 2H), 1.43 (s, 3H), 1.25 (s, 3H).

### Example 5: Preparation of (S)-2-((((Z)-1-(2-aminothiazol-4-yl)-2-(((S)-2,2-dimethyl-4-oxo-1-(sulfonyloxy)azetidin-3-yl)amino)-2-oxoethylene)amino)oxy)-3-(4-(6-((S)-2,6-diamino hexanamido)pyridin-3-yl)phenoxy)propionic acid

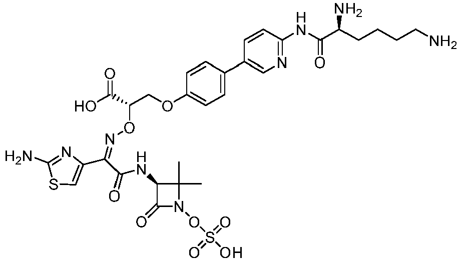

The synthesis in Example 5 is similar to that in Example 4, except that step 5 was omitted to give the title compound. ESI-MS m/z:764.2 [M+H]⁺. ¹H NMR (400 MHz, DMSO*-*d₆+D₂O) δ 8.59 - 8.50 (m, 1H), 8.18 - 8.01 (m, 2H), 7.64 - 7.53 (m, 2H), 7.06 - 6.91 (m, 2H), 6.87 (s, 1H), 4.89 - 4.79 (m, 1H), 4.73 (s, 1H), 4.47 - 4.34 (m, 1H), 4.32 - 4.23 (m, 1H), 4.11 - 4.03 (m, 1H), 2.91 - 2.72 (m, 2H), 1.92 - 1.74 (m, 2H), 1.69 - 1.53 (m, 2H), 1.46 (s, 3H), 1.44 - 1.38 (m, 2H), 1.29 -1.25 (m, 3H).

For the synthesis methods of Examples 6, 7, 8, and 9, refer to Example 4.

| Examples | Structures | ESI-MS, ¹H NMR |
|---|---|---|
| 6 | 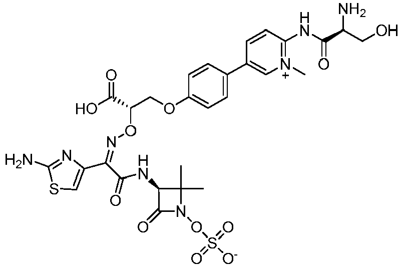 | ESI-MS m/z:737.1 [M+H]⁺ |
| | | ¹H NMR (400 MHz, DMSO*-d₆*+D₂O) δ 8.52 (s, 1H), 8.29 - 8.17 (m, 2H), 7.65 (d, 2H), 7.08 (d, 2H), 6.85 (s, 1H), 4.94 (s, 1H), 4.68 (s, 1H), 4.39 (s, 2H), 3.96 - 3.85 (m, 5H), 3.77 (s, 1H), 1.42 (s, 3H), 1.19 (s, 3H). |
| 7 | 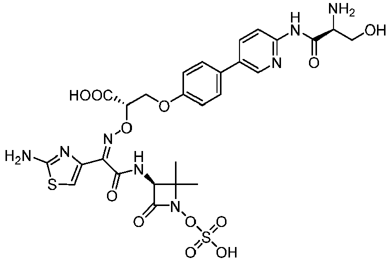 | ESI-MS m/z:723.1 [M+H]⁺ |
| | | ¹H NMR (400 MHz, DMSO*-d₆*+D₂O) δ 8.62 (d, 1H), 8.19 - 8.08 (m, 2H), 7.64 (t, 2H), 7.18 - 7.02 (m, 2H), 6.82 (d, 1H), 4.78 (d, 1H), 4.69 (d, 1H), 4.36 (d, 2H), 3.98 (s, 1H), 3.80 (s, 2H), 1.43 (s, 3H), 1.25 (s, 3H). |
| 8 | 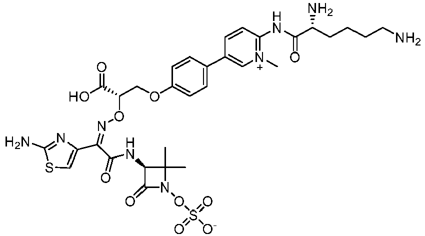 | ESI-MS m/z:778.2 [M+H]⁺ |
| | | ¹H NMR (400 MHz, DMSO*-d₆*+D₂O) δ 8.47 (s, 1H), 8.22 (d, *J* = 9.3 Hz, 1H), 8.13 (d, *J* = 9.2 Hz, 1H), 7.55 (d, *J* = 7.8 Hz, 2H), 7.03 (d, *J* = 7.9 Hz, 2H), 6.83 (s, 1H), 4.85 - 4.71 (m, 1H), 4.69 (s, 1H), 4.39 - 4.23 (m, 2H), 3.85 (s, 3H), 3.70 - 3.63 (m, 1H), 2.86 - 2.70 (m, 2H), 1.94 - 1.73 (m, 2H), 1.64 - 1.51 (m, 2H), 1.50 - 1.42 (m, 2H), 1.41 (s, 3H), 1.23 (s, 3H). |
| 9 | 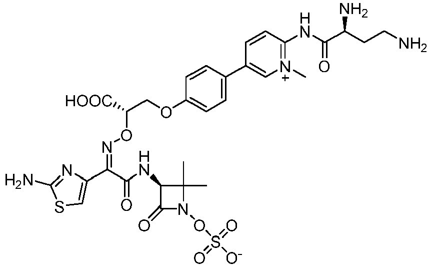 | ESI-MS m/z:750.2 [M+H]⁺ |
| | | ¹H NMR (400 MHz, DMSO*-d₆*+D₂O) δ 8.39 (s, 1H), 8.28 - 8.19 (m, 1H), 8.11 (s, 1H), 7.46 (d, *J* = 7.3 Hz, 2H), 6.97 (d, *J* = 7.2 Hz, 2H), 6.86 (s, 1H), 4.78 (s, 1H), 4.71 (s, 1H), 4.37 - 4.22 (m, 2H), 3.95 (s, 1H), 3.84 (s, 3H), 3.20 - 2.96 (m, 2H), 2.34 - 1.98 (m, 2H), 1.44 (s, 3H), 1.26 (s, 3H). |

### Example 10: (S)-3-((Z)-2-(((S)-4-(4-(1-(3-aminopropyl)-2-(azetidin-3-ylmethyl)-1H-pyrazol-2-ium-4-yl)phenoxy)-1-carboxybutoxy)imino)-2-(2-aminothiazol-4-yl)acetylamino)-2,2-dimethyl-4-oxoazetidin-1-yl sulfate

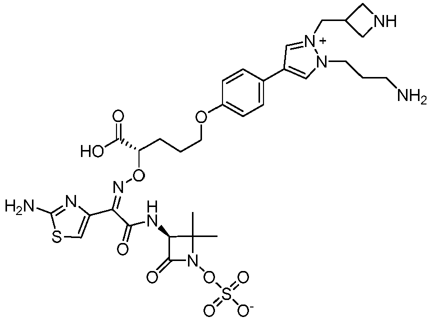

### Step 1: Preparation of (S)-2,2-dimethyl-1,3-dioxolane-4-carbaldehyde

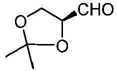

Dry 4A molecular sieve (76 g), pyridinium chlorochromate (49 g, 0.23 mol) and anhydrous dichloromethane (400ml) were added sequentially to a reaction flask. (*R*)-(2,2-dimethyl-1,3-dioxolan-4-yl)methanol (20 g, 0.15 mol) was added while controlling the temperature with an ice bath. After the addition, the mixture was allowed to naturally warm to room temperature and the reaction was carried out overnight. When the raw materials disappeared, the reaction was complete. Post-treatment: The mixture was filtered through a pad of celite, and the filtrate was concentrated at 20°C under reduced pressure to give a brown semi-solid, which was not weighed, and was completely used in the next step.

### Step 2: Preparation of ethyl (R)-3-(2,2-dimethyl-1,3-dioxolane-4-yl)acrylate

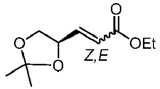

The crude product of the previous step (theoretically 19.7 g, 0.15 mol, calculated as 100% conversion), ethyl 2-(diethoxyphosphoryl)acetate (34 g, 0.15 mol), potassium carbonate (20.9 g, 0.15 mol), water (300 ml) and tetrahydrofuran (100 ml) were added sequentially, and reacted overnight at room temperature until the reaction was complete. Post-treatment: The mixture was extracted twice with dichloromethane, and the combined dichloromethane phase was washed once with saturated sodium chloride, dried over anhydrous sodium sulfate, concentrated with silica gel, and subjected to column chromatography to give 7.2 g of colorless oil.

### Step 3: Preparation of ethyl (R)-3-(2,2-dimethyl-1,3-dioxolan-4-yl)propionate

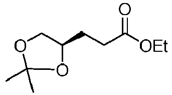

Ethyl (*R*)-3-(2,2-dimethyl-1,3-dioxolan-4-yl)acrylate (7.2 g), palladium on carbon (720 mg) and methanol (50 ml) were added sequentially. After purging 3 times with hydrogen, the reaction was carried out at room temperature overnight until the reaction was complete. Post-treatment: filtering, washing palladium carbon with methanol, and concentrating the filtrate to give 5.15 g of colorless oil, which was completely used in the next step. ¹H NMR (400 MHz, Chloroform-*d*) δ 4.17 - 4.09 (m, 3H), 4.05 (dd, 1H), 3.55 (dd, 1H), 2.52 - 2.33 (m, 2H), 1.96 - 1.79 (m, 2H), 1.41 (s, 3H), 1.34 (s, 3H), 1.26 (t, 3H).

### Step 4: Preparation of (R)-3-(2,2-dimethyl-1,3-dioxolan-4-yl)propan-1-ol

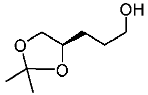

A solution of ethyl (*R*)-3-(2,2-dimethyl-1,3-dioxolan-4-yl)propionate (5.15 g, 25.5 mmol) in anhydrous tetrahydrofuran (25 ml) was added slowly and dropwise into a suspension of lithium aluminum hydride (1.26 g, 33.1 mmol) at -10°C, while controlling the temperature at lower than -5°C. After the addition, the mixture was allowed to naturally warm to room temperature to react for 4 h. When the raw materials disappear, the reaction was complete. Post-treatment: The mixture was cooled to -10°C. Water (2.4 ml) was added dropwise to quench the reaction, after which the mixture was warmed to room temperature. Dichloromethane (150 ml) was added, and the organic phase was dried over anhydrous sodium sulfate, filtered and concentrated to give 4.4 g of the title compound. ¹H NMR (400 MHz, Chloroform-*d*) δ 4.18 - 4.09 (m, 1H), 4.08 - 4.04 (m, 1H), 3.66 - 3.10 (m, 2H), 3.55 - 3.52 (m, 1H), 1.74 - 1.60 (m, 4H), 1.43 (d, *J* = 4.7 Hz, 3H), 1.37 (s, 3H).

### Step 5: Preparation of (R)-3-(2,2-dimethyl-1,3-dioxolan-4-yl)propyl-4-toluenesulfonate

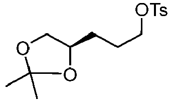

(*R*)-3-(2,2-dimethyl-1,3-dioxolan-4-yl)propan-1-ol (4.4 g, 27.5 mmol) was dissolved in a solution of p-toluenesulfonyl chloride (5.76 g, 30.3 mmol) and triethylamine (4.17 g, 41.3 mmol) in dichloromethane (80 ml), and reacted overnight at room temperature. When the raw materials disappeared, the reaction was completed. Post-treatment: Saturated ammonium chloride was added to quench the reaction. The mixture was extracted with dichloromethane. The organic phase was dried over anhydrous sodium sulfate, concentrated, and subjected to column chromatography to give 7.87 g of the title compound.

### Step 6: Preparation of (R)-4-(3-(4-bromophenoxy)propyl)-2,2-dimethyl-1,3-dioxolane

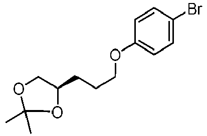

(*R*)-3-(2,2-dimethyl-1,3-dioxolan-4-yl)propyl-4-toluenesulfonate (7.87 g, 25.1 mmol), p-bromophenol (4.34 g, 25.1 mmol), potassium carbonate (5.21 g, 37.6 mmol) and *N,N-*dimethylformamide (80 ml) were added sequentially. The reaction was carried out at 100°C for 2 h. When the raw materials disappeared, the reaction was complete. Post-treatment: The reaction was quenched with water. The mixture was extracted with ethyl acetate. The organic phase was washed with saturated brine, dried over anhydrous sodium sulfate, filtered, concentrated, and subjected to column chromatography to give 7.60 g of the title compound. ESI-MS m/z: 315.0 [M+H]⁺.

### Step 7: Preparation of (R)-5-(4-bromophenoxy)pentane-1,2-diol

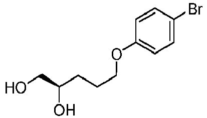

(*R*)-4-(3-(4-bromophenoxy)propyl)-2,2-dimethyl-1,3-dioxolane (7.60 g, 24.20 mmol) was dissolved in a mixed solution of 1 mol/L hydrochloric acid solution (1 mol/L, 40 ml) and tetrahydrofuran (40 ml). The mixture was stirred at room temperature for 1.5 hours until the reaction was complete. Post-treatment: The mixture was cooled in an ice bath, adjusted to about pH 7 with saturated solution of sodium bicarbonate, and extracted twice with ethyl acetate. The combined ethyl acetate phase was dried over anhydrous sodium sulfate, filtered, concentrated, and subjected to column chromatography to give 5.66 g of colorless oil. ESI-MS m/z: 275.0 [M+H]⁺.

### Step 8: Preparation of tert-butyl (R)-5-(4-bromophenoxy)-2-hydroxyvalerate

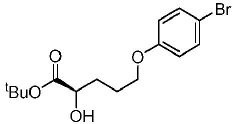

The title compound was prepared by a preparation method which was similar to the preparation method of steps 13 and 14 in Example 1, except that the raw material (*S*)-3-(4-bromophenoxy)- 1,2-propanediol was replaced with (*R*)-5-(4-bromophenoxy)pentan-1,2-diol.

### Step 9: preparation of tert-butyl (3-(4-(4,4,5,5-tetramethyl-1,3,2-dioxaborolan-2-yl)-1H-pyrazol-1-yl)propyl)carbamate

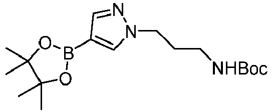

Tert-butyl (3-bromopropyl)carbamate (4.42 g, 18.55 mmol) was added to a mixed liquid of 4-(4,4,5,5-tetramethyl-1,3,2-dioxaborolan-2-yl)-1*H*-pyrazole (3 g, 15.46 mmol) and cesium carbonate (7.58 g, 23.19 mmol), and reacted overnight at room temperature. After the raw materials disappeared, water was added to quench the reaction. The mixture was extracted with ethyl acetate, and the organic phase was washed with saturated brine, dried over anhydrous sodium sulfate, filtered, concentrated, and subjected to column chromatography to give the title compound.

### Step 10: Preparation of (S)-3-((Z)-2-(((S)-4-(4-(1-(3-aminopropyl)-2-(azetidin-3-ylmethyl)-1H-pyrazol-2-ium-4-yl)phenoxy)-1-carboxybutoxy)imino)-2-(2-aminothiazol-4-yl)acetylamino)-2,2 -dimethyl-4-oxoazetidin-1-yl sulfate

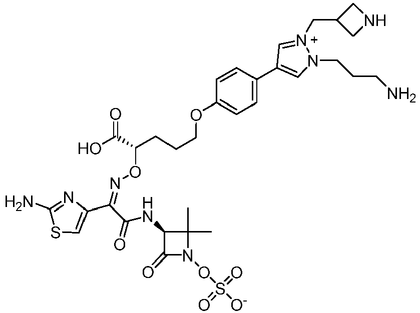

The title compound was prepared by a preparation method which was similar to the preparation method of steps 3-9 in Example 4, except that tert-butyl (*R*)-3-(4-bromophenoxy)-2- hydroxypropionate was replaced with tert-butyl (*R*)-5-(4-bromophenoxy)-2-hydroxyvalerate, the di(tert-butyl) (6-oxo-6-((5-(4,4,5,5-tetramethyl-1,3,2-dioxaborolan-2-yl)pyridin-2-yl)amino) hexan-1,5-diyl)(*S*)-dicarbamate was replaced with tert-butyl (3-(4-(4,4,5,5-tetramethyl-1,3,2-dioxaborolan-2-yl)-1*H*-pyrazol-1-yl)propyl)carbamate, and methyl iodide was replaced with tert-butyl 3-((((trifluoromethyl)sulfonyl) oxy)methyl)azetidine-1-carboxylate. ESI-MS m/z:764.3 [M+H]⁺, ¹H NMR (400 MHz, D₂O) δ 8.61 (s, 1H), 8.50 (s, 1H), 7.50 (d, *J* = 8.5 Hz, 2H), 6.96 (d, *J* = 8.5 Hz, 2H), 6.73 (s, 1H), 4.78 - 4.75 (m, 2H), 4.54 - 4.48 (m, 2H), 4.48 - 4.44 (m, 1H), 4.31 - 4.19 (m, 2H), 4.10 - 3.99 (m, 5H), 3.68 - 3.50 (m, 1H), 3.11 - 3.03 (m, 2H), 2.37 - 2.21 (m, 2H), 1.88 - 1.78 (m, 2H), 1.78 - 1.63 (m, 2H), 1.23 (s, 3H), 1.05 (s, 3H).

### Example 11: (S)-3-(4-(2-(aminomethyl)-1H-benzo[d]imidazol-5-yl)phenoxy)-2-((((Z)-1-(2-aminothiazol-4-yl)-2-(((S)-2,2-dimethyl-4-oxo-1-(sulfonyloxy)azetidin-3-yl)amino)-2-oxoethyle ne)amino)oxy)propionic acid

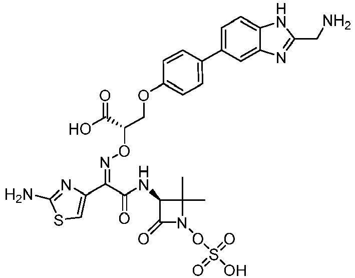

### Step 1: Preparation of tert-butyl (2-((2-amino-4-bromophenyl)amino)-2-oxoethyl)carbamate

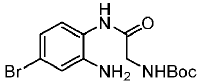

4-Bromo-o-phenylenediamine (5.61 g, 30 mmol) and tert-butoxycarbonylglycine (5.25 g, 30 mmol) were dissolved in dichloromethane (50 ml), followed by sequentially adding 2-(7-oxobenzotriazolyl)-*N,N,N',N'*-tetramethyluronium hexafluorophosphate (13.6 g, 36 mmol) and *N,N*-diisopropylethylamine (90 mmol). The reaction was carried out at room temperature overnight until the raw materials disappeared completely. Post-treatment: Water was added for extraction, and the aqueous phase was extracted twice with dichloromethane. The combined organic phase was dried over anhydrous sodium sulfate. Dichloromethane was removed under reduced pressure, and the residue was purified by column chromatography to give 8 g of a white solid. ESI-MS m/z:344.0 [M+H]⁺.

### Step 2: Preparation of tert-butyl ((5-bromo-1H-benzo[d]imidazol-2-yl)methyl)carbamate

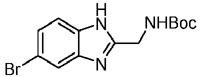

Tert-butyl (2-((2-amino-4-bromophenyl)amino)-2-oxoethyl)carbamate (8 g, 23.3 mmol) was dissolved in acetic acid (50 ml). The mixture was heated to 65°C and reacted for 5 h until the raw materials disappeared completely. Post-treatment: The mixture was rotary evaporated to dryness to remove acetic acid, and the residue was directly purified by column chromatography to give 3.2 g of a yellow solid. ESI-MS m/z:326.0 [M+H]⁺.

### Step 3: Preparation of tert-butyl (R)-2-hydroxy-3-(4-(4,4,5,5-tetramethyl-1,3,2-dioxaborolan-2-yl) phenoxy)propionate

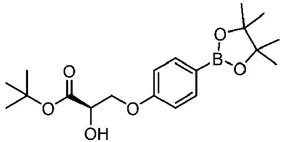

The title compound was prepared by a preparation method which was similar to step 2 of Example 4, except that di(tert-butyl) (6-((5-bromopyridin-2-yl)amino)-6-oxohexan-1,5-diyl) (S)-dicarbamate was replaced with tert-butyl (*R*)-3-(4-bromophenoxy)-2-hydroxypropionate.

### Step 4: Preparation of tert-butyl (R)-3-(4-(2-(((tert-butoxycarbonyl)amino)methyl)-1H-benzo[d]imidazol-5-yl)phenoxy)-2-hydroxypropionate

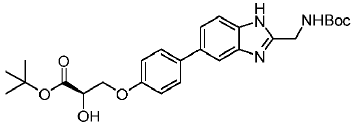

Tert-butyl ((5-bromo-1*H*-benzo[*d*]imidazol-2-yl)methyl)carbamate (1 g, 3.08 mmol) and tert-butyl (*R*)-2-hydroxy-3-(4-(4,4,5,5-tetramethyl-1,3,2-dioxaborolan-2-yl)phenoxy)propionate (1.12 g, 3.08 mmol) were dissolved in a mixed solvent of dioxane (10 ml) and water (3 ml), followed by adding a catalyst (200 mg, 0.30 mmol) and sodium carbonate (980 mg, 9.23 mmol). The system was purged and protected with argon. The mixture was heated to 100°C, and reacted for 2 h. After the raw materials disappeared and the reaction was complete, the mixture was cooled to room temperature, followed by adding water. The mixture was extracted with ethyl acetate, and the combined organic phase was purified by column chromatography to give 520 mg of the product. ESI-MS m/z:484.2 [M+H]⁺.

### Step 5: Preparation of (S)-3-(4-(2-(aminomethyl)-1H-benzo[d]imidazol-5-yl)phenoxy)-2-((((Z)-1-(2-aminothiazol-4-yl)-2-(((S)-2,2-dimethyl-4-oxo-1-(sulfonyloxy)azetidin-3-yl)amino)-2-oxoethylene)amino)oxy)propionic acid

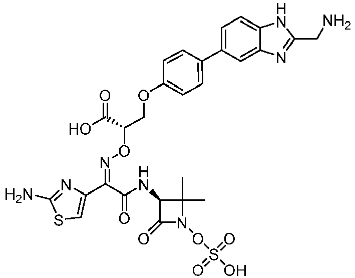

The title compound was prepared by a preparation method which was similar to the preparation method of steps 4 and 6-9 in Example 4, except that the raw material tert-butyl (*R*)-3-(4-(6-((*S*)-2,6-bis((tert-butoxycarbonyl)amino)hexanamido)pyridin-3-yl)phenoxy)-2-hydr oxypropionate was replaced with tert-butyl (*R*)-3-(4-(2-(((tert-butoxycarbonyl)amino)methyl)-1*H-*benzo[d]imidazol-5-yl)phenoxy)-2-hydroxy propionate. ESI-MS m/z:689.3 [M+H]⁺. ¹H NMR (400 MHz, DMSO*-d₆*+D₂O) δ 8.62 (d, 1H), 8.19 - 8.08 (m, 2H), 7.64 (t, 2H), 7.18 - 7.02 (m, 2H), 6.82 (d, 1H), 4.78 (d, 1H), 4.69 (d, 1H), 4.36 (d, 2H), 3.80 (s, 2H), 1.43 (s, 3H), 1.25 (s, 3H).

### Example 12: 3-(2-(((S)-2-((1-(3-aminopropyl)-2-methyl-1H-pyrazol-2-ium-4-yl)oxy)-1-carboxy ethoxy)imino)-2-(2-aminothiazol-4-yl)acetylamino)-2,2-dimethyl-4-oxoazetidin-1-yl sulfate

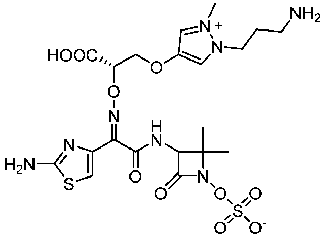

### Step 1: preparation of tert-butyl (3-(4-(4,4,5,5-tetramethyl-1,3,2-dioxaborolan-2-yl)-1H-pyrazol-1-yl)propyl)carbamate

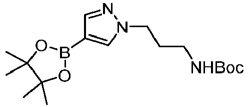

4-(4,4,5,5-Tetramethyl-1,3,2-dioxaborolan-2-yl)-1*H*-pyrazole (4 g, 20.6 mmol), tert-butyl (3-bromopropyl)carbamate (4.9 g, 20.6 mmol) and cesium carbonate (10.08 g, 30.9 mmol) were dissolved in dimethylformamide (100 ml), and reacted overnight at room temperature, until the raw materials disappeared and the reaction was complete. Post-treatment: Water was added for extraction, and the aqueous phase was extracted twice with ethyl acetate. The combined organic phase was dried over anhydrous sodium sulfate. Ethyl acetate was removed under reduced pressure, and the residue was purified by column chromatography to give 7 g of the product as a white solid. ESI-MS m/z: 352.2 [M+H]⁺.

### Step 2: Preparation of tert-butyl (3-(4-hydroxy-1H-pyrazol-1-yl)propyl)carbamate

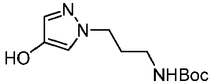

Tert-butyl (3-(4-(4,4,5,5-tetramethyl-1,3,2-dioxaborolan-2-yl)-1*H*-pyrazol-1-yl)propyl)carbamate (7 g, 19.9 mmol), sodium hydroxide (2 mol/L, 20 ml) and hydrogen peroxide (30%, 20 ml) were dissolved in tetrahydrofuran (50 ml), and reacted at room temperature for 1 h, until the raw materials disappeared and the reaction was complete. Post-treatment: Water was added for extraction, and the aqueous phase was extracted twice with ethyl acetate. The combined organic phase was dried over anhydrous sodium sulfate. Ethyl acetate was removed under reduced pressure, and the residue was purified by column chromatography to give 3.922 g of the product as a pale yellow solid. ESI-MS m/z: 242.2 [M+H]⁺.

### Step 3: Preparation of 3-(2-(((S)-2-((1-(3-aminopropyl)-2-methyl-1H-pyrazol-2-ium-4-yl)oxy)-1-carboxyethoxy)imino)-2-(2-aminothiazol-4-yl)acetylamino)-2,2-dimethyl-4-oxoazetidin-1-yl sulfate

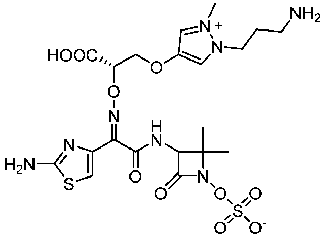

The title compound was prepared by a preparation method which was similar to the preparation method of steps 4 and 6-9 in Example 4, except that the raw material tert-butyl (*R*)-3-(4-(6-((*S*)-2,6-bis((tert-butoxycarbonyl)amino)hexanamido)pyridin-3-yl)phenoxy)-2-hydr oxypropionate was replaced with tert-butyl (3-(4-hydroxy-1*H*-pyrazol-1-yl)propyl)carbamate. ESI-MS m/z: 605.3 [M+H]⁺. ¹H NMR (400 MHz, DMSO*-d₆*+D₂O) δ 8.39 (d, *J* = 5.5 Hz, 2H), 6.82 (s, 1H), 4.52-4.43 (m, 2H), 4.41-4.29 (m, 2H), 4.05 (s, 3H), 2.92-2.79 (m, 2H), 2.18-2.05 (m, 2H), 1.42 (s, 3H), 1.25-1.21 (m, 5H).

According to the synthesis method of Example 1 of the present invention, compounds of Examples 13-20 were synthesized from different commercially available raw materials. The characterization parameters of these compounds are shown in Table 1.

**Table 1:**

| Examples | Structures of the compounds | ESI-MS m/z [M+H]⁺ |
|---|---|---|
| 13 | 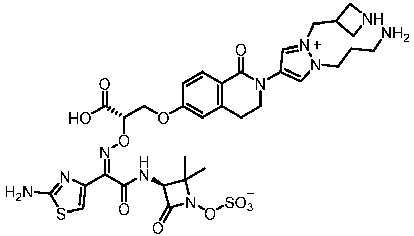 | 804.9 |
| 14 | 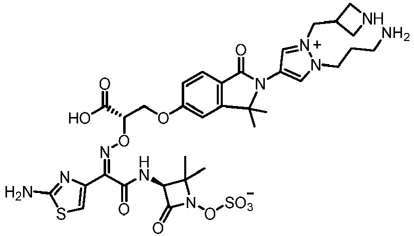 | 818.9 |
| 15 | 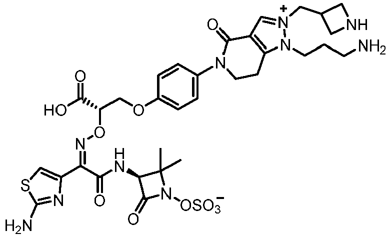 | 804.9 |
| 16 | 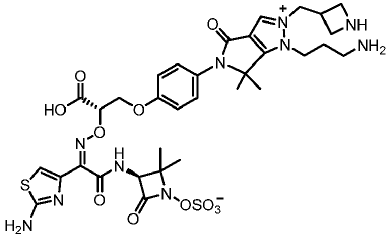 | 818.9 |
| 17 | 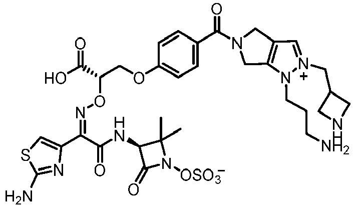 | 804.9 |
| 18 | 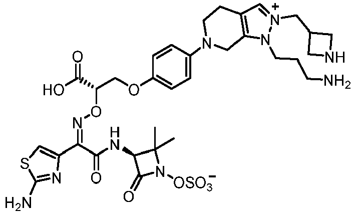 | 790.9 |
| 19 | 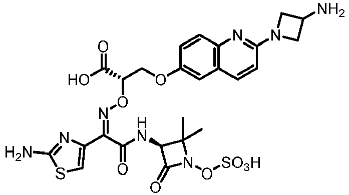 | 664.7 |
| 20 | 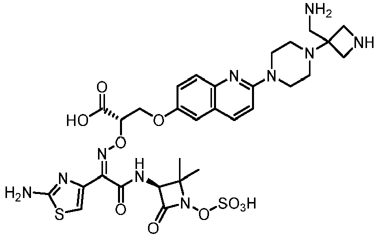 | 762.8 |
| 21 | 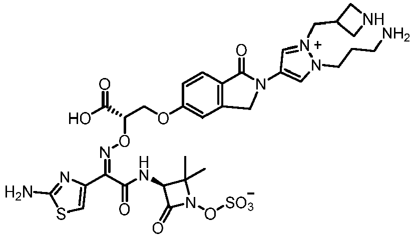 | 790.8 |
| 22 | 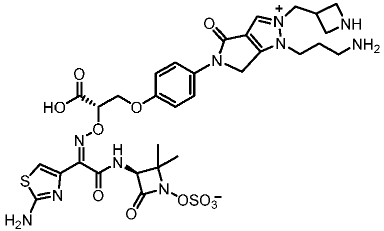 | 790.8 |
| 23 | 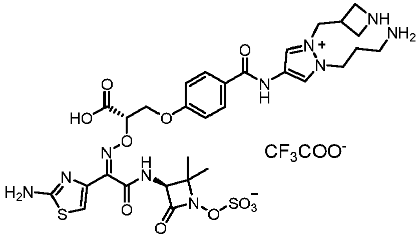 | 779.9 |
| 24 | 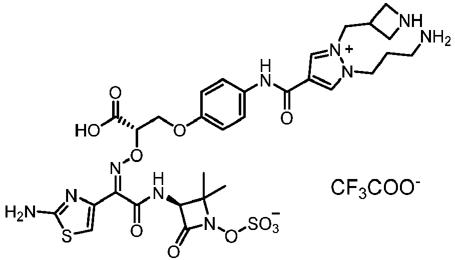 | 779.9 |
| 25 | 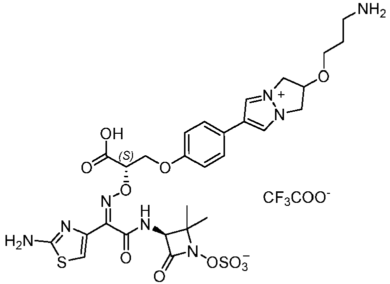 | 722.6 |
| 26 | 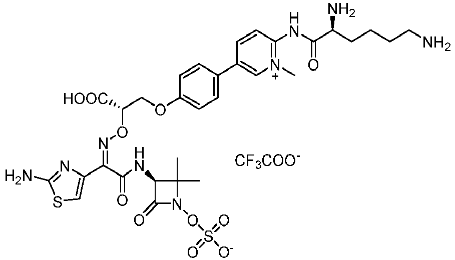 | 778.2 |
| 27 | 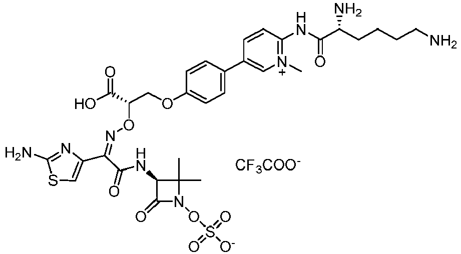 | 778.2 |
| 28 | 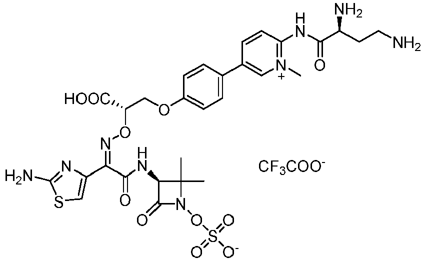 | 750.2 |
| 29 | 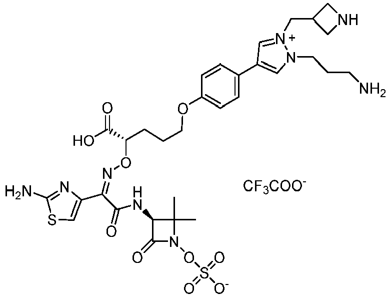 | 764.3 |

### Comparative Example 1

According to the method disclosed in Example 76 in WO2017/106064 (PCT/US2016/066064), the compound represented by the following formula (Compound A) was prepared and characterized by ¹H-NMR and mass spectrometry,

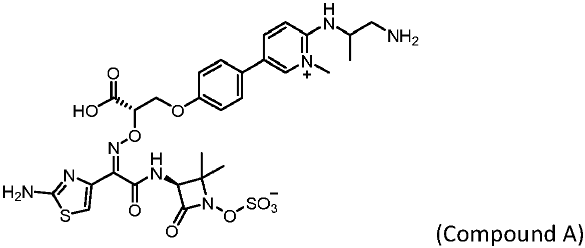

The in vitro antibacterial activity of Compound A was tested using the method of Experimental Example 1 below. The experimental results show that Compound A, which shows the best inhibition to Acinetobacter baumannii in WO2017/106064, has significantly weaker in vitro antibacterial activity than some compounds of the present invention.

### Comparative Example 2

According to the method disclosed in Example 22 of WO2015/148379 (PCT/US2015/022011), the compound represented by the following formula (Compound B) was prepared and characterized by ¹H-NMR and mass spectrometry,

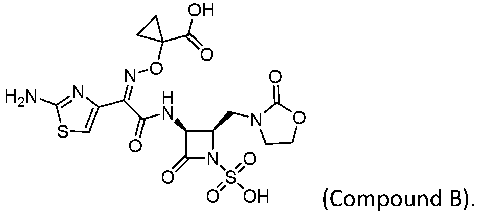

The in vitro antibacterial activity of Compound B was tested using the method of Experimental Example 1 below. The experimental results show that Compound B has significantly weaker in vitro antibacterial activity than some compounds of the present invention.

### Comparative Example 3

According to the method disclosed in Example 97 of WO2017/106064 (PCT/US2016/066064), the compound represented by the following formula (Compound C) was prepared and characterized by ¹H-NMR and mass spectrometry,

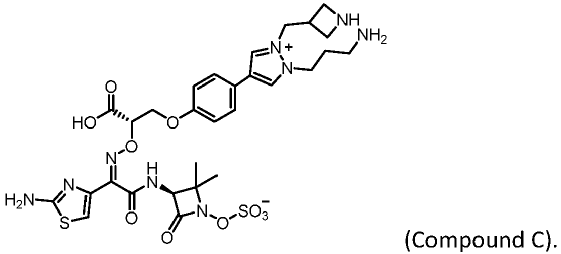

The in vitro antibacterial activity and pharmacokinetic characteristics of Compound C were tested using the methods of the following Experimental Examples 1 and 2. The experimental results show that Compound C has weaker in vitro antibacterial activity and bioavailability (F) than some compounds of the present invention.

### Experimental Example 1: Evaluation of in vitro antibacterial activity of compounds

### 1. Experimental materials

Compounds: The compounds prepared in the above examples and comparative examples. All test compounds were dissolved and diluted with DMSO, and the final concentrations were prepared as 64, 32, 16, 8, 4, 2, 1, 0.5, 0.25, 0.125, 0.0625 µg/mL in the experiment.

Bacterial strains: 6 strains of Acinetobacter baumannii (codenames: HDBAB-YK4, HDBAB-YK7, HDBAB-YK12, HDBAB-YK16, HDBAB_OXA_7, ATCC 19606), 2 strains of Pseudomonas aeruginosa (codenames: HDBPA-YK9, HDBPA)-YK15), 1 strain of Escherichia coli (codename: CLB30048), all provided by HD Biosciences (Shanghai) Co., Ltd.

Medium: Trypticase soy agar (TSA) (BD BBL 211043), and Cation-adjusted Mueller Hinton broth (CAMHB) (BD BBL212322).

### 2. Experimental method

### 2.1. Recovery of bacteria

The bacteria used for the minimum inhibitory concentration (MIC) test were stored frozen at -80°C, and needed 2 days to recover before use. A small amount of frozen bacteria was scraped with a sterile inoculation loop for streak inoculation on a TSA solid medium petri dish. The dish was placed in an ordinary incubator at 35±2°C for 20-24 hours. 5-10 Colonies with similar morphologies were picked from the above-mentioned petri dish with a sterile inoculating loop for streak inoculation again on a TSA solid medium petri dish. The dish was then placed in an ordinary incubator at 35±2°C for 20-24 hours.

### 2.2. Preparation for bacterial inoculation

1.02X CAMHB liquid medium was taken out from a 4°C refrigerator and placed at room temperature for preheating. 5-10 Individual bacterial colonies were picked from the solid petri dish of the above Section 2.1 and re-suspended in 500 µL of 0.9% NaCl, and then the OD600 was adjusted to 0.1-0.15 with a spectrophotometer. Then the bacteria were diluted 400 times with 1.02X CAMHB (two-step dilution method: firstly diluting 10 times, then diluting 40 times).

The number of inoculated bacteria can be obtained by counting the colonies on the petri dish (see Section 2.4).

### 2.3. Preparation of test plate

### 2.3.1 Arrangement of test plate

One compound was arranged in each row of a 96-well test plate: the highest test concentration for each compound was 64 µg/mL, which was diluted by a factor of two.

Growth control (GC): 1.02X CAMHB and dimethyl sulfoxide (DMSO) containing bacterial inoculum, no compound.

Sterile control (SC): Containing 1.02X CAMHB and DMSO, no compound.

### 2.3.2 Compound dilution

All test compounds were dissolved and diluted with DMSO. 50 µL of 3.2 mg/mL of each test compound was transferred to the initial well (A1 - H1) of the dilution plate, and then 25 µL of DMSO was transferred to other wells. The test compounds were sequentially diluted by a factor of 2 from column 1 to column 11 (i.e. drawing 25 µL of test compound from column 1 to column 2 and mixing well, and then drawing 25 µL of test compound from column 2 to column 3 and mixing well, and then drawing 25 µL of test compound from column 3 to column 4 and mixing well, and so on, until diluting to column 11).

2 µL of the compound was transferred from the compound plate to the corresponding well of the test plate. Meanwhile, 2 µL of 100% DMSO was transferred to the compound-free wells (the GC and SC wells).

98 µL of the corresponding bacterial inoculum was added to the test plate (except for the SC well).

98 µL of 1.02X CAMHB medium was added to the SC well of the test plate.

After the system was added, the test plate was covered with a sterile lid, centrifuged at 800 rpm for 30 seconds, then shaken on a plate shaker at 400 rpm for 1 minute for mixing well, and then placed in an incubator at 35±2°C for 20 hours.

### 2.4. Colony counts

The inoculated bacteria were gradient diluted by 10 folds with the liquid medium, from 10⁻¹ to 10⁻³. 100 µL of the above bacterial dilution was spread evenly on a TSA petri dish, 2 replicates for each dilution. After the medium was absorbed by TSA for 10 minutes, the petri dishes were reversed and incubated in an incubator at 35±2°C for 24 hours.

### 2.5. Recording of minimum inhibitory concentration (MIC) and statistics of colonies

A compound management system was turned on to check whether the barcode and compound arrangement of each test plate were correct.

The test plate was placed on the plate reading device and the reflector was adjusted to observe and record the growth of the bacteria in each well. At the same time, pictures were taken for each test plate with the QCount system.

The minimum inhibitory concentration (MIC) of each compound was recorded according to the guidelines of the Clinical and Laboratory Standards Institute.

The number of colonies of different dilutions of bacterial inoculum in the TSA petri dish was counted and the amount of bacterial inoculation was calculated.

### 3. Experimental results

The minimum inhibitory concentration (MIC) results for some compounds are shown in Table 2.

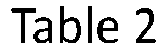

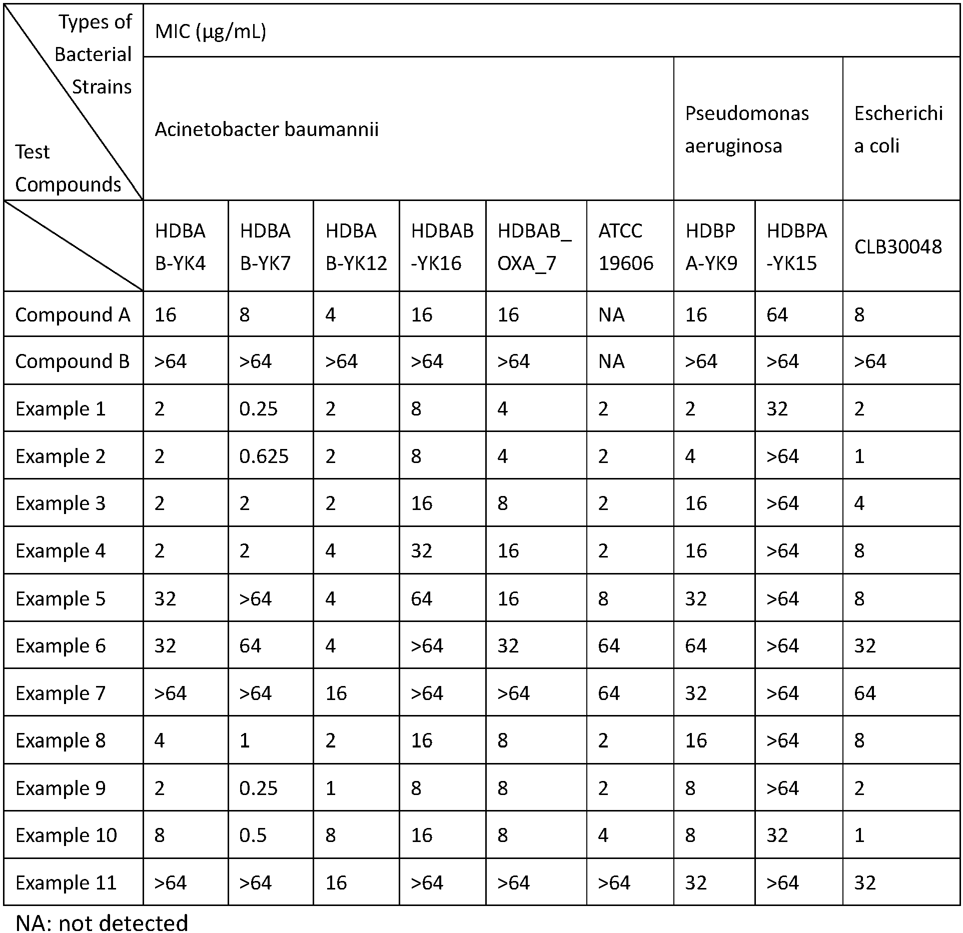

It can be seen from the above experimental results that the minimum inhibitory concentrations (MICs) of some compounds of the present invention against Acinetobacter baumannii, Pseudomonas aeruginosa, and Escherichia coli can be as low as 2 µg/mL or less. For example, the compounds of Example 1, Example 2, Example 3, and Example 9 showe excellent antibacterial activity.

### Experimental Example 2: Evaluation of pharmacokinetics of the compounds in mice

### 1. Experimental materials

Animals: male BALB/c mice, SPF grade, purchased from Changzhou Cavens Experimental Animal Co., Ltd.; 16-24 g, license number: SCXK (Su)2016-0010; 2 to 3 days of adaptation period were given before the experiment.

Instruments: API 4500 High Performance Liquid Chromatograph Coupled Triple Quadrupole Mass Spectrometer, and Analyst QS A01.01 Chromatography Workstation were both purchased from AB SCIEX of the United States; Milli-Q ultrapure water device was purchased from Millipore; CF16R XII desktop high-speed refrigerated centrifuge was purchased From Hitachi Company; Qilinbeier Vortex-5 oscillator was purchased from IKA Company of German; electric heating constant-temperature water bath was purchased from Changzhou Guohua Electric Appliance Co., Ltd.; electric pipette was purchased from Thermo Company of American; and microanalysis balance was purchased from Shanghai Mettler Co., Ltd.

### 2. Experimental method

### 2.1 Preparation of test drugs

2 mg of the test compound was weighed, added to physiological saline, vortexed for 2 min, and ultrasonicated for 3 min, to prepare a test solution with a concentration of 0.2 mg/mL for intravenous administration.

### 2.2 Sample collection

A single dose of 2 mg/kg of the test compound was administered to BALB/c mice via the tail vein, the dose volume being 0.1 mL/10 g. At 5 min, 15 min, 30 min, 1 h, 2 h, 4 h after administration, blood samples were collected from the orbital venous plexus into heparinized EP tubes (0.6 mL), which were temporarily placed on crushed ice.

### 2.3 Sample processing and analysis

The samples were centrifuged at 8000 rpm for 5 min. 15 µL of upper plasma were transferred to a 96-well plate. 150 µL of methanol:acetonitrile (v/v = 1:1) (containing 20 ng/mL tolbutamide) was added to 15 µL of plasma, shaken for 3 min, and centrifuged at 4500 rpm for 5 min. 100 µL of supernatant was taken to a 2 mL deep well plate. 100 µL of diluent (pure water) was added. The samples were shaken for 3 min, and centrifuged at 4500 rpm for 5 min. 180 µL of the supernatant was transferred to the sample plate. The content of the compound in the supernatant sample was analyzed by LC-MS/MS, and various pharmacokinetic parameters were calculated with WinNonlin software.

### 3. Experimental results

The pharmacokinetic parameters of some compounds are shown in Table 3.

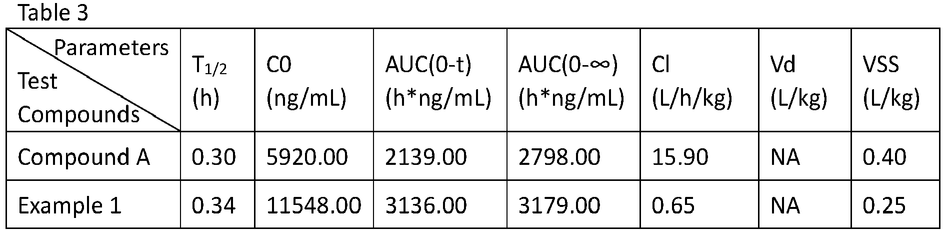

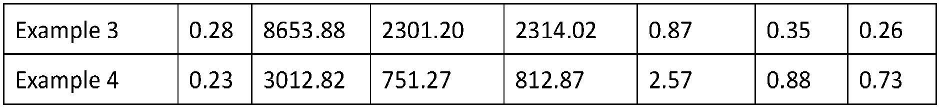

It can be seen from the above experiments that the compound of Example 1 of the present invention has a blood concentration of 11.548 µg/mL (C0) at a intravenous dose of 2 mg/kg, which is much higher than the MIC of the compound, indicating that a dose of 2 mg/kg is sufficient to achieve excellent antibacterial effects.

### Experimental example 3: MIC experiment of clinically sourced bacterial strains

Compounds: the compound of Example 1 and Compound C of Comparative Example 3. Both test compounds were dissolved and diluted with DMSO, and the final concentrations were prepared as 64, 32, 16, 8, 4, 2, 1, 0.5, 0.25, 0.125, 0.0625 µg/mL in the experiment.

Bacterial strains: clinically isolated Acinetobacter baumannii, Pseudomonas aeruginosa, Klebsiella pneumoniae and Escherichia coli, all provided by HD Biosciences (Shanghai) Co., Ltd.

Medium: Trypticase soy agar (TSA) (BD BBL 211043), and Cation-adjusted Mueller Hinton broth (CAMHB) (BD BBL212322).

The experimental method was the same as that in the above Experimental Example 1. The experimental results are shown in Table 4.

**Table 4**

| Types of Bacterial Stains | Namecodes of Bacterial Strains | MIC of Compound of Example 1 (µg/mL) | MIC of Compound C (µg/mL) |
|---|---|---|---|
| *Acinetobacter baumannii* | A0242702 | ≤0.063 | ≤0.063 |
| | A0244122 | 0.5 | 2 |
| | A0248164 | 1 | 2 |
| | A0248384 | 1 | 2 |
| | A0252247 | ≤0.063 | 0.125 |
| | A0254384 | ≤0.063 | 0.125 |
| | A0255830 | 4 | 16 |
| | A0257674 | ≤0.063 | 0.125 |
| | A0258522 | ≤0.063 | 0.125 |
| | A0262855 | ≤0.063 | 0.125 |
| | A0270386 | 1 | 1 |
| | A0272260 | 0.125 | 0.125 |
| | A0273375 | 1 | 1 |
| | A0273826 | 4 | 16 |
| | A0274925 | 0.125 | 0.25 |
| | A0283307 | ≤0.063 | 0.125 |
| | A0284214 | ≤0.063 | 0.125 |
| | A0284295 | ≤0.063 | 0.125 |
| | A0284552 | 0.5 | 0.5 |
| | A0285002 | 1 | 2 |
| | A0285050 | 0.125 | 0.25 |
| | A0285231 | 1 | 2 |
| | A0285299 | ≤0.063 | 0.25 |
| | A0285369 | ≤0.063 | ≤0.063 |
| | A0285909 | 4 | 16 |
| | A0286065 | 1 | 2 |
| *Pseudomonas aeruginosa* | P7 | 1 | 2 |
| | P43 | 0.25 | 0.25 |
| | P155 | 1 | 2 |
| | P157 | 1 | 2 |
| | P162 | 1 | 2 |
| *Klebsiella pneumoniae* | Kp309 | ≤0.063 | 0.25 |
| | Kp310 | ≤0.063 | ≤0.063 |
| | Kp472 | 0.125 | 0.125 |
| | Kp510 | 0.25 | 0.25 |
| *Escherichia coli* | HDB0140 | 1 | 2 |
| | HDB5510 | 0.125 | 0.25 |
| | HDB8244 | 0.5 | 0.25 |
| | HDB1120 | ≤0.063 | 0.125 |
| | HDB0918 | 0.25 | 0.25 |

It can be seen from the above experiments that the compound of Example 1 has excellent antibacterial activities against clinically isolated Acinetobacter baumannii, Pseudomonas aeruginosa, Klebsiella pneumoniae and Escherichia coli, and the effects on various strains were significantly better than those of Compound C. For example, the MIC values of the compound of Example 1 in respect of A0244122, A0255830, A0273826, A0285299, A0285909 and Kp309 are only 1/4 of those of Compound C. At the same time, the compound of Example 1 shows MIC ≤ 0.063 µg/mL (the lowest detection concentration) in respect of 11 strains, while Compound C shows MIC ≤ 0.063 µg/mL in respect of only 2 strains, indicating that the compound of Example 1 is significantly better than Compound C. In addition, it was experimentally determined that the MIC₉₀ value of the compound of Example 1 of the present invention against clinically isolated Acinetobacter baumannii is 4 µg/mL, while the MIC₉₀ value of Compound C against clinically isolated Acinetobacter baumannii is 16 µg/mL. The MIC₉₀ value of the compound of Example 1 is only a quarter of that of Compound C, indicating that the compound of Example 1 is significantly better than Compound C.

In addition, the inventors of the present invention also tested the inhibitory activities of the compound of Example 1 and Compound C, respectively, in combination with Relebactam on drug-resistant Acinetobacter baumannii (the experimental method was the same as that in Experimental Example 1). The experimental results show that for drug-resistant Acinetobacter baumannii HDBAB-YK12, HDBAB-YK16 and HDBAB_OXA_7, the MIC values of the compound of Example 1 in combination with 4 µg/mL Relebactam are 2 µg/mL, 4 µg/mL, 2 µg/mL, respectively, while the MIC values of compound C in combination with 4 µg/mL Relebactam are 8 µg/mL, 16 µg/mL and 16 µg/mL, respectively. It can be seen that the antibacterial activity of the compound of Example 1 is significantly better than that of Compound C.

Although the present invention has been described above in detail, those skilled in the art would understand that various modifications and changes can be made without departing from the spirit and scope of the present invention. The claimed scope of the present invention is not limited to the detailed description made above, but should be defined by the claims.

## Claims

1. A compound represented by general formula (I), or an isomer, pharmaceutically acceptable salt, solvate, crystal or prodrug thereof, 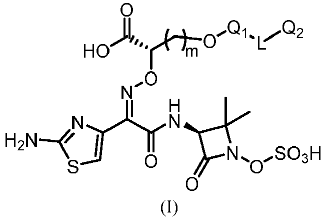 wherein
Q₁ is selected from the group consisting of aryl, aryl-fused-heterocyclyl, heteroaryl-fused-heterocyclyl, 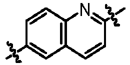 and 5-membered heteroaryl, which is optionally substituted by one or more R¹;
Q2 is absent or is selected from the group consisting of 4-6 membered heterocyclyl, aryl-fused-heterocyclyl, heteroaryl-fused-cycloalkyl, aryl-fused-heteroaryl, heteroaryl-fused-heterocyclyl, 5-membered heteroaryl and 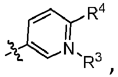 which is optionally substituted by one or more R², R³ is absent or is R², and R⁴ is selected from the group consisting of aminoalkylamido and hydroxyaminoalkylamido;
R¹ is selected from the group consisting of halogen, hydroxy, alkyl, haloalkyl, hydroxyalkyl, alkoxy, haloalkoxy, hydroxyalkoxy, nitro, carboxy, cyano, amino, monoalkylamino, alkylamido, alkylacyl, carbamoyl, alkylcarbamoyl, dialkylamino, alkenyl, alkynyl, cycloalkyl, heterocyclyl, aryl, heteroaryl and oxo;
R² is selected from the group consisting of halogen, hydroxy, alkyl, haloalkyl, hydroxyalkyl, aminoalkyl, alkoxy, haloalkoxy, hydroxyalkoxy, aminoalkoxy, nitro, carboxy, cyano, amino, monoalkylamino, alkylamido, aminoalkylamido, hydroxyaminoalkylamido, alkylacyl, carbamoyl, alkylcarbamoyl, dialkylamino, alkenyl, alkynyl, cycloalkyl, heterocyclyl, heterocyclylalkyl, aminoheterocyclyl, aminoalkylheterocyclyl, hydroxyalkylheterocyclyl, aryl, heteroaryl and oxo, which is optionally substituted by amino, aminoalkyl, alkylamino, halogen, hydroxy, alkyl, haloalkyl, hydroxyalkyl, alkoxy, haloalkoxy, hydroxyalkoxy, aminoalkoxy, nitro, carboxyl or cyano;
L is absent or is selected from the group consisting of -C(O)NH-, -NHC(O)-, -SO₂NH-, -NHSO₂, -(CH₂)ₙ- and -C(O)-;
m is 1, 2, 3 or 4;
n is 1, 2, 3 or 4; and
when Q₁ is aryl, Q₂ is selected from the group consisting of aryl-fused-heterocyclyl, heteroaryl-fused-cycloalkyl, aryl-fused-heteroaryl and heteroaryl-fused-heterocyclyl; or
when Q₁ is aryl, Q₂ is 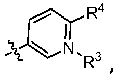 wherein R³ is absent or is R², and R⁴ is selected from the group consisting of aminoalkylamido and hydroxyaminoalkylamido; or
when Q₁ is aryl, Q₂ is 5-membered heteroaryl, and L is selected from the group consisting of -C(O)NH-, -NHC(O)-, -SO₂NH-, -NHSO₂-, -(CH₂)ₙ- and -C(O)-; or
when Q₁ is aryl, Q₂ is 5-membered heteroaryl, L is absent, and m is 2, 3 or 4; or
when Q₁ is selected from the group consisting of aryl-fused-heterocyclyl and heteroaryl-fused-heterocyclyl, Q₂ is 5-membered heteroaryl, and L is absent; or
when Q₁ is 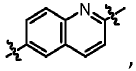 L is absent, and Q2 is 4-6 membered heterocyclyl substituted by R²; or
when Q₁ is 5-membered heteroaryl, Q₂ is absent, and L is absent.

2. The compound according to claim 1, or an isomer, pharmaceutically acceptable salt, solvate, crystal or prodrug thereof, wherein
Q₁ is selected from the group consisting of 6-18 membered aryl, 9-20 membered aryl-fused-heterocyclyl, 9-20 membered heteroaryl-fused-heterocyclyl, 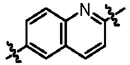 and 5-membered heteroaryl, which is optionally substituted by one or more R¹;
Q2 is absent or is selected from the group consisting of 4-6 membered heterocyclyl, 9-20 membered aryl-fused-heterocyclyl, 9-20 membered heteroaryl-fused-cycloalkyl, 9-20 membered aryl-fused-heteroaryl, 9-20 membered heteroaryl-fused-heterocyclyl, 5-membered heteroaryl and 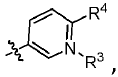 which is optionally substituted by one or more R², R³ is absent or is R², R⁴ is selected from the group consisting of aminoC₁₋₁₂alkylamido and hydroxyaminoC₁₋₁₂alkylamido;
R¹ is selected from the group consisting of halogen, hydroxy, C₁₋₆alkyl, haloC₁₋₆alkyl, hydroxyC₁₋₆alkyl, C₁₋₆alkoxy, haloC₁₋₆alkoxy, hydroxyC₁₋₆alkoxy, nitro, carboxy, cyano, amino, monoC₁₋₆alkylamino, C₁₋₆alkylamido, C₁₋₆alkylacyl, carbamoyl, C₁₋₆alkylcarbamoyl, diC₁₋₆alkylamino, C₂₋₁₀alkenyl, C₂₋₁₀alkynyl, C₃₋₁₂cycloalkyl, 3-12 membered heterocyclyl, 6-12 membered aryl, 5-12 membered heteroaryl and oxo; and
R² is selected from the group consisting of halogen, hydroxy, C₁₋₆alkyl, haloC₁₋₆alkyl, hydroxyC₁₋₆alkyl, aminoC₁₋₆alkyl, C₁₋₆alkoxy, haloC₁₋₆alkoxy, hydroxyC₁₋₆alkoxy, aminoC₁₋₆alkoxy, nitro, carboxy, cyano, amino, monoC₁₋₆alkylamino, C₁₋₆alkylamido, aminoC₁₋₁₂alkylamido, hydroxyaminoC₁₋₁₂alkylamido, C₁₋₆alkylacyl, carbamoyl, C₁₋₆alkylcarbamoyl, diC₁₋₆alkylamino, C₂₋₁₀alkenyl, C₂₋₁₀alkynyl, C₃₋₁₂cycloalkyl, 3-12 membered heterocyclyl, 3-12 membered heterocyclylC₁₋₆alkyl, amino 3-12 membered heterocyclyl, aminoC₁₋₆alkyl 3-12 membered heterocyclyl, hydroxyC₁₋₆alkyl 3-12 membered heterocyclyl, 6-12 membered aryl, 5-12 membered heteroaryl and oxo, which is optionally substituted by amino, aminoC₁₋₆alkyl, C₁₋₆alkylamino, halogen, hydroxy, C₁₋₆alkyl, haloC₁₋₆alkyl, hydroxyC₁₋₆alkyl, C₁₋₆alkoxy, haloC₁₋₆alkoxy, hydroxyC₁₋₆alkoxy, aminoC₁₋₆alkoxy, nitro, carboxy or cyano.

3. The compound according to claim 1 or 2, or an isomer, pharmaceutically acceptable salt, solvate, crystal or prodrug thereof, wherein the general formula (I) has the structure of the following general formula (lb), 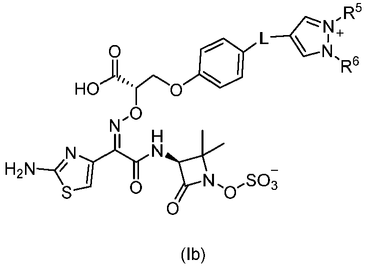 wherein
L is selected from the group consisting of -C(O)NH-, -NHC(O)-, -SO₂NH-, -NHSO₂, -(CH₂)ₙ- and -C(O)-; and
R⁵ and R⁶ are each independently selected from the group consisting of halogen, hydroxy, alkyl, haloalkyl, hydroxyalkyl, aminoalkyl, alkoxy, haloalkoxy, hydroxyalkoxy, aminoalkoxy, nitro, carboxy, cyano, amino, monoalkylamino, alkylamido, aminoalkylamido, hydroxyaminoalkylamido, alkylacyl, carbamoyl, alkylcarbamoyl, dialkylamino, alkenyl, alkynyl, cycloalkyl, heterocyclyl, heterocyclylalkyl, aminoheterocyclyl, aminoalkylheterocyclyl, hydroxyalkylheterocyclyl, aryl, heteroaryl and oxo, which is optionally substituted by amino, aminoalkyl, alkylamino, halogen, hydroxy, alkyl, haloalkyl, hydroxyalkyl, alkoxy, haloalkoxy, hydroxyalkoxy, aminoalkoxy, nitro, carboxy or cyano.

4. The compound according to claim 3, or an isomer, pharmaceutically acceptable salt, solvate, crystal or prodrug thereof, wherein
L is selected from the group consisting of -C(O)NH- and -NHC(O)-; and
R⁵ and R⁶ are each independently selected from the group consisting of halogen, hydroxy, C₁₋₆alkyl, haloC₁₋₆alkyl, hydroxyC₁₋₆alkyl, aminoC₁₋₆alkyl, C₁₋₆alkoxy, haloC₁₋₆alkoxy, hydroxyC₁₋₆alkoxy, aminoC₁₋₆alkoxy, nitro, carboxy, cyano, amino, monoC₁₋₆alkylamino, C₁₋₆alkylamido, aminoC₁₋₆alkylamido, hydroxyaminoC₁₋₆alkylamido, C₁₋₆alkylacyl, carbamoyl, C₁₋₆alkylcarbamoyl, diC₁₋₆alkylamino, C₂₋₆alkenyl, C₂₋₆alkynyl, C₃₋₈cycloalkyl, 3-8 membered heterocyclyl, 3-8 membered heterocyclylC₁₋₆alkyl, amino 3-8 membered heterocyclyl, aminoC₁₋₆alkyl 3-8 membered heterocyclyl, hydroxyC₁₋₆alkyl 3-8 membered heterocyclyl, aryl, 3-8 membered heteroaryl and oxo, which is optionally substituted by amino, aminoC₁₋₆alkyl, C₁₋₆alkylamino, halogen, hydroxy, C₁₋₆alkyl, haloC₁₋₆alkyl, hydroxyC₁₋₆alkyl, C₁₋₆alkoxy, haloC₁₋₆alkoxy, hydroxyC₁₋₆alkoxy, aminoC₁₋₆alkoxy, nitro, carboxy or cyano.

5. The compound according to claim 3 or 4, or an isomer, pharmaceutically acceptable salt, solvate, crystal or prodrug thereof, wherein R⁶ is selected from the group consisting of

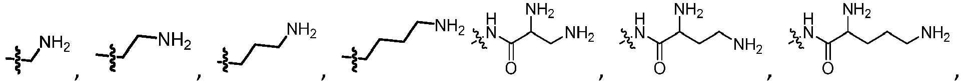

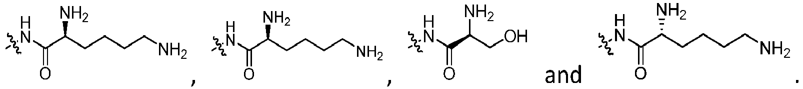

6. The compound according to any one of claims 3-5, or an isomer, pharmaceutically acceptable salt, solvate, crystal or prodrug thereof, wherein R⁵ is selected from the group consisting of methyl, ethyl, propyl, cyclopropyl,

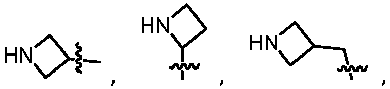

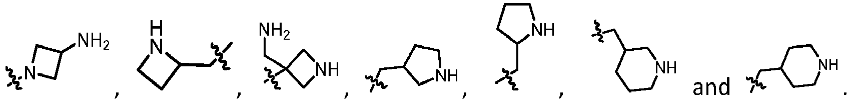

7. The compound according to claim 1 or 2, or an isomer, pharmaceutically acceptable salt, solvate, crystal or prodrug thereof, wherein the general formula (I) has the structure of the following general formula (Ia), 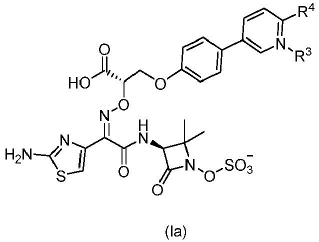 wherein
R³ is absent or is R²; and
R⁴ is selected from the group consisting of aminoalkylamido and hydroxyaminoalkylamido; preferably selected from the group consisting of amino aminoC₁₋₆alkylamido and hydroxyaminoC₁₋₆alkylamido; more preferably selected from the group consisting of

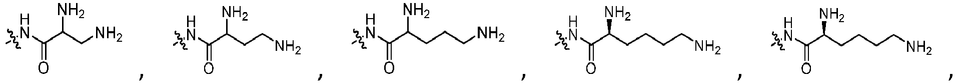

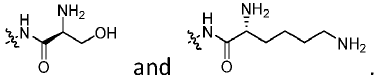

8. The compound according to claim 1, or an isomer, pharmaceutically acceptable salt, solvate, crystal or prodrug thereof, wherein the compound is a compound selected from the group consisting of: 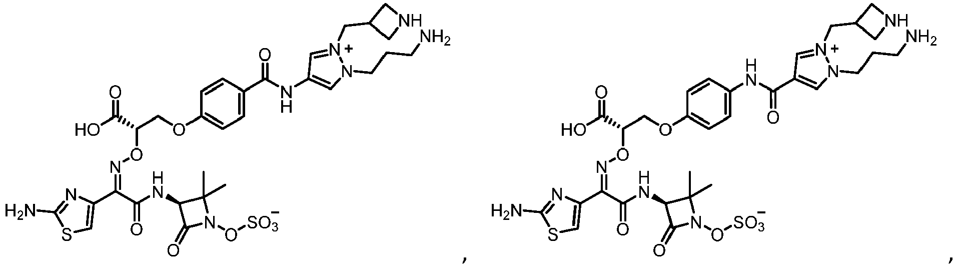 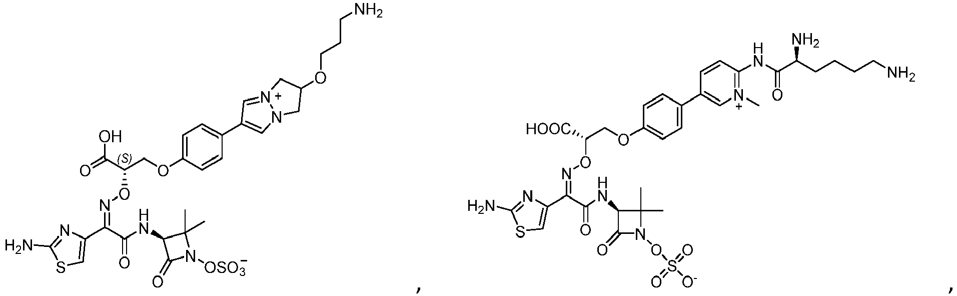 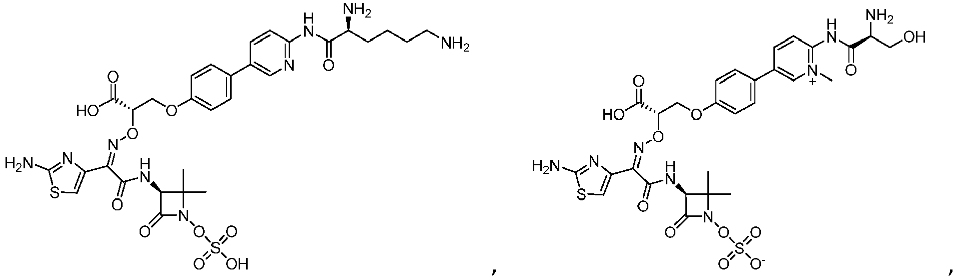 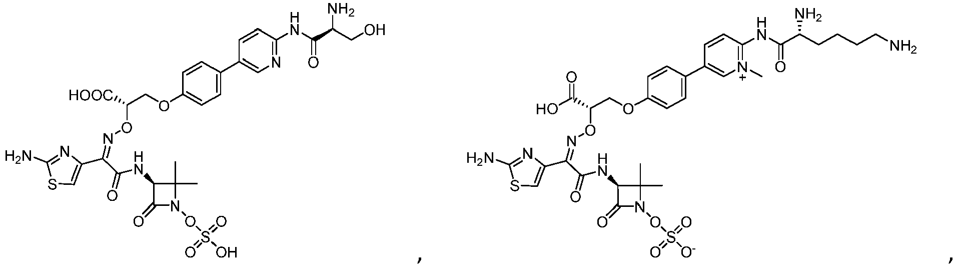 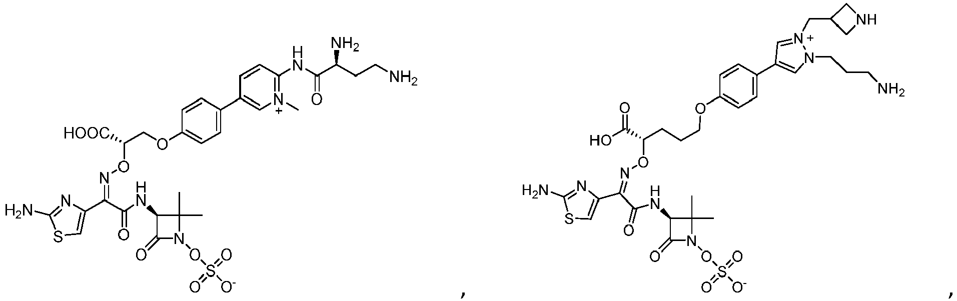 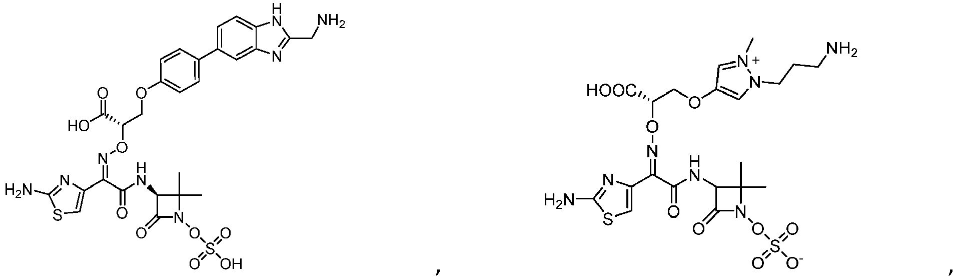 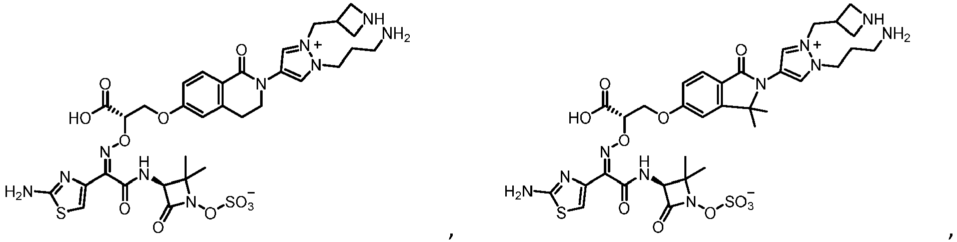 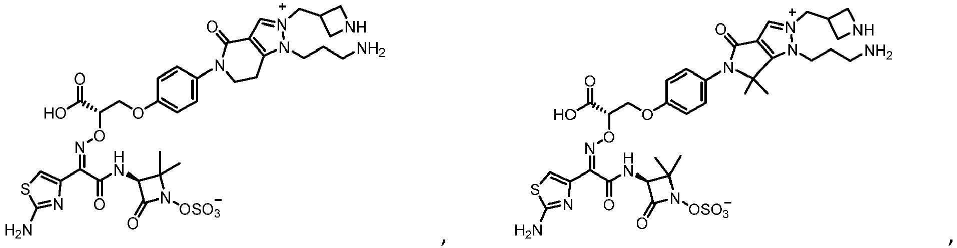 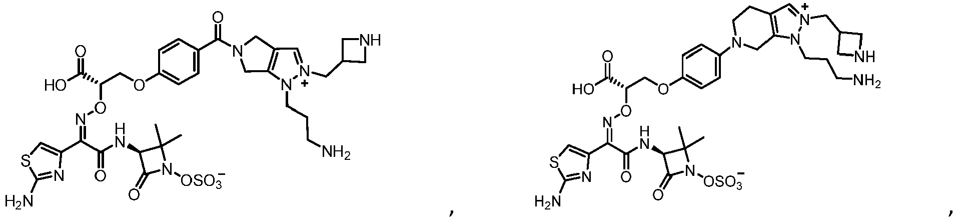 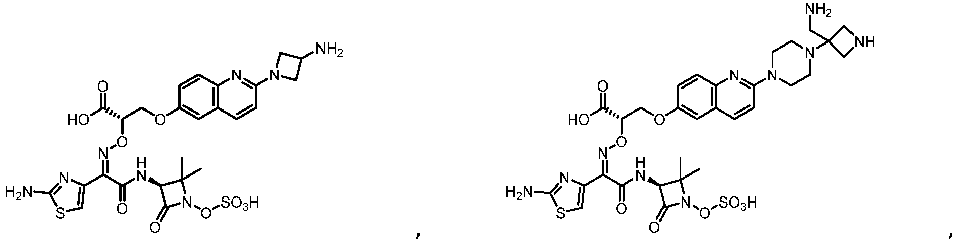 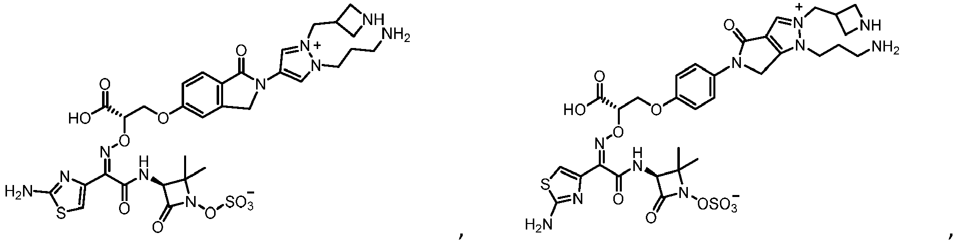 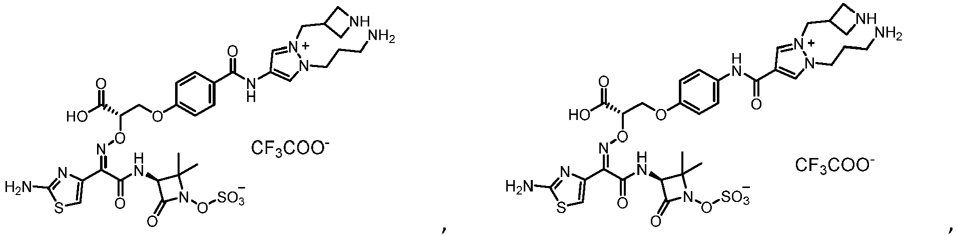 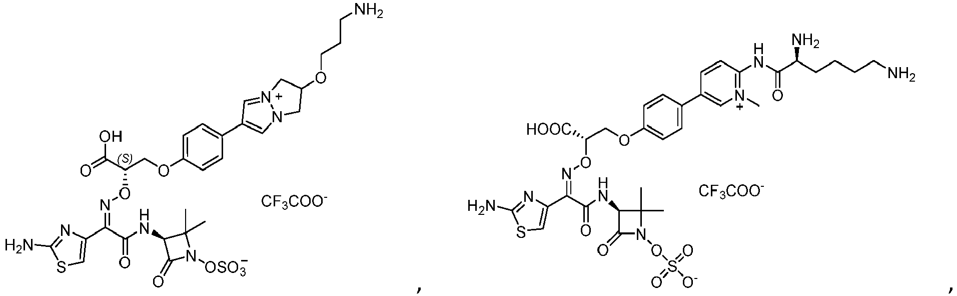 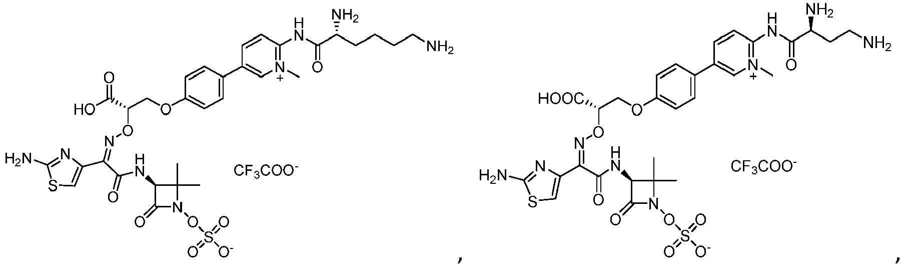 and

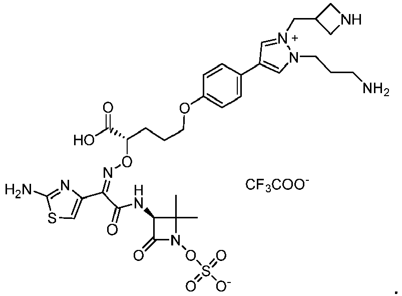

9. A pharmaceutical composition comprising the compound according to any one of claims 1 to 8, or an isomer, pharmaceutically acceptable salt, solvate, crystal or prodrug thereof, and a pharmaceutically acceptable carrier.

10. Use of the compound according to any one of claims 1-8, or an isomer, pharmaceutically acceptable salt, solvate, crystal or prodrug thereof, or the pharmaceutical composition according to claim 9 in the manufacture of a medicament for the treatment of a bacterial infection.
